# EUROPEAN PATENT SPECIFICATION

(11) **EP 3 022 340 B1**
(45) Date of publication and mention of the grant of the patent: **16.09.2020**
(21) Application number: 14825858.5
(22) Date of filing: 18.07.2014
(51) Int. Cl.: B01J 19/00, C07K 1/04, G01N 33/50, C40B 50/14, C40B 40/10, C40B 30/00

(54) **PARALLEL ORGANIC SYNTHESIS ON PATTERNED PAPER USING A SOLVENT-REPELLING MATERIAL**
PARALLELE ORGANISCHE SYNTHESE AUF STRUKTURIERTEM PAPIER UNTER ANWENDUNG EINES LÖSUNGSMITTELABWEISENDEN MATERIALS
SYNTHÈSE ORGANIQUE PARALLÈLE SUR PAPIER À MOTIF À L'AIDE D'UN MATÉRIAU REPOUSSANT LES SOLVANTS

(30) Priority: 18.07.2013 US 201361847647 P; 27.03.2014 US 201461970998 P
(43) Date of publication of application: 25.05.2016
(73) Proprietor: The Governors of the University of Alberta, Edmonton, Alberta T5J 4P6 (CA)
(72) Inventor: DERDA, Ratmir, Edmonton, Alberta T5K 0H2 (CA); DEISS, Frederique, Edmonton, Alberta T5J 4P6 (CA)
(74) Representative: Appleyard Lees IP LLP
(86) International application number: PCT/CA2014/050683
(87) International publication number: WO 2015/006874

(56) References cited:
- WO-A2-02/094846
- US-B2- 7 005 294
- ERICA UEDA ET AL: "DropletMicroarray: facile formation of arrays of microdroplets and hydrogel micropads for cell screening applications", LAB ON A CHIP, 1 January 2012 (2012-01-01), XP055043855, ISSN: 1473-0197, DOI: 10.1039/c2lc40921f
- FRÉDÉRIQUE DEISS ET AL: "Platform for High-Throughput Testing of the Effect of Soluble Compounds on 3D Cell Cultures", ANALYTICAL CHEMISTRY, vol. 85, no. 17, 12 July 2013 (2013-07-12) , pages 8085-8094, XP055336162, US ISSN: 0003-2700, DOI: 10.1021/ac400161j
- DEISS ET AL.: 'FLOW-THROUGH SYNTHESIS ON TEFLON-PATTERNED PAPER TO PRODUCE PEPTIDE ARRAYS FOR CELL-BASED ASSAYS.' ANGEWANDTE CHEMIE INTERNATIONAL EDITION vol. 53, no. 25, 2014, pages 6374 - 6377, XP055310955
- KOZAK ET AL.: 'Micro-volume wall-less immunoassays using patterned planar plates.' LAB CHIP vol. 13, no. 7, 2013, pages 1342 - 1350, XP055310959
- DIRK F.H. WINKLER.: 'Chemistry of SPOT synthesis for the preparation of peptide macroarrays on cellulose membranes.' MINI-REVIEWS IN ORGANIC CHEMISTRY vol. 8, 2011, pages 114 - 120, XP008181969
- CATHERINE J. WU. ET AL.: 'SPOT synthesis as a tool to study protein-protein interactions (by Winkler et al.). Protein microarray for disease analysis: Methods and protocols.' METHODS IN MOLECULAR BIOLOGY vol. 723, 2011, pages 105 - 127, XP008181968
- CARRILHO ET AL.: 'Understanding wax printing: A simple micropatterning process for paper-based microfluidics.' ANALYTICAL CHEMISTRY vol. 81, no. 16, 2009, pages 7091 - 7095, XP002585317
- HILPERT ET AL.: 'Peptide arrays on cellulose support: SPOT synthesis, a time and cost efficient method for synthesis of large numbers of peptides in a parallel and addressable fashion.' NATURE PROTOCOLS vol. 2, no. 6, 2007, pages 1333 - 1349, XP009157029
- HILPERT ET AL.: 'Cellulose-bound peptide arrays: Preparation and applications.' BIOTECHNOLOGY AND GENETIC ENGINEERING REVIEWS vol. 24, no. 1, 2007, pages 31 - 106, XP055310968
- HEINE ET AL.: 'Synthesis and screening of peptoid arrays on cellulose membranes.' TETRAHEDRON vol. 59, no. 50, 2003, pages 9919 - 9930, XP004474435
- REINEKE ET AL.: 'Applications of peptide arrays prepared by the SPOT-technology.' CURRENT OPINION IN BIOTECHNOLOGY vol. 12, no. 1, 2001, pages 59 - 64, XP002234261
- RONALD FRANK.: 'Spot-synthesis: An easy technique for the positionally addressable, parallel chemical synthesis on a membrane support.' TETRAHEDRON vol. 48, no. 42, 1992, pages 9217 - 9232, XP026636781

## Description

### FIELD

The present application pertains to organic molecular synthesis. In particular, the present application relates to chemical modification of paper and synthesis of complex organic molecules on paper.

### BACKGROUND

In the field of combinatorial array synthesis -- such as SPOT synthesis on paper and other parallel syntheses on the planar supports -- large collections of spatially immobilized molecules are useful for applications such as biomolecule discovery, discovery of drug leads and mapping of the functional parts of biomolecules (epitope mapping).

Chemical modification of cellulose is an essential step for the development of new value-added, cellulose-based products. The modification of cellulose, and other porous materials, equips cellulose with novel abilities such as, but not limited to, the ability to retain certain chemicals (chemisorption, filtration), its ability to deactivate pathogens, and decreased or increased permeability of paper to moisture, paints, adhesives of other modifying agents.

In the field of diagnostics, control of liquid flow in one sheet of paper is important for multiplexing the detection and processing of the reagent. The benefits of confinement of aqueous solutions, known as paper microfluidics, for these diagnostic applications are characterized in the art (see, for example, US 20090298191 A1). One deficiency in this field is the confinement of a non-aqueous solution, such as ethanol, which can be required in some sample processing steps; analogously, it is problematic to retain aqueous solutions that contain reagents that could destroy the pattern. Examples are synthetic surfactants present in the processing solution or natural surfactants and amphiphilic molecules present in the biological fluids.

Patterning of paper by hydrophobic patterns to retain water and govern flow of aqueous solutions is known in the art. Examples are SU8, PDMS and wax printing (see US patent publication 20090298191 A1) and patterning of thermoplastic and thermoreactive polymers via "sweet patterning" (European patent application EP 2265959 A2). These patent documents describe general patterning methods, they do not describe producing an oleophobic barrier that could provide long-term confinement of organic solvents. The patterns described therein may be divided into two classes, each with a specific deficiency: Class I barriers that are not chemically stable to organic solvents; examples are wax barriers and barriers made of thermoplastic organic polymers (polystyrene, etc); and Class II barriers which are materials that do not react with organic solvent - these are represented by covalently cross-linked polymers, such as PDMS and SU8 - but exhibit low contact angle with most organic solvents. Such a low contact angle prevents effective confinement of organic solvents needed for the purposes of organic synthesis and would not provide significant benefits over non-patterned paper.

The coating of substrates such as paper sheets or cellulose pulp with fluorochemicals to impart grease, oil, wax and solvent repellency are known in the art. These are referred to as "bulk modifications". Examples of bulk modifications are described in Schwartz, in "Oil Resistance Utilizing Fluorochemicals," TAPPI, Seminar Notes, 74, 71-75, (1987), who discloses the use of fluorochemicals to render various substrates oil resistant. The improvements in the art describing fluorochemical compositions for imparting oil and water repellency to various substrates are described in US Patent 5,674,961 US Patent 5,330,622, U.S. Patent No. 4,426,466, U.S. Patent No. 4,529,658, and U.S. Patent No. 5,370,919. These patents do not describe millimiter-scale patterning of fluorochemicals. Extension of bulk modification of porous material to patterned deposition of fluorochemicals on these materials is non-obvious based on the existing art.

Parallel synthesis on paper is known in the art. This concept has been developed to yield commercial products such as arrays of peptides immobilized in spatially-defined locations on a sheet of paper. Exemplary products are provided by companies such as: Intavis, Kinexus, Pepmetric, Genscript, JPT, activeMotif, or Sigma-Aldrich(Genesys). This synthesis is based on non-patterned paper. To create the array, small amounts of solutions of the reagents are deposited in precise locations on the paper. It is critical to deposit limited amount of liquids per unit area to allow spatial arrangements of different chemicals on paper and prevent their spreading and mixing. One fundamental problem with supported synthesis is the delivery of the sufficient quantity of the reagent to the support. As liquids spread on paper by capillary action, a unit area of paper can hold only a limited amount of solvent approximately defined as V₀=S×h×p, where h is the height, s is the area and p is the porosity. It has typically been difficult to deposit more than V₀ of the solution per unit area because the liquid spreads to a larger area to maintain the ratio (volume)/(surface) constant. Confinement of the solvent by solvophobic border increases the (volume)/(surface) ratio by a factor of 3-50 and enables supported synthesis with new solvents (e.g. those with low vapour pressure) and new chemical transformations (e.g. those that require large excess of reagent).

One existing solution to maintain increased amount of solvent per unit area is encasing paper into a metal holder (one example is provided by Intavis Corporation). Metal frame-based confinement has numerous disadvantages, including inflexibility, lack of ability to be stacked, and the presence of wicks on the inside.

Synthesis on a solid support requires optimal reaction conditions in which the transport of the reagent to the immobilized reactive sites is faster than the rate of the reaction. Such conditions are best achieved in an agitated reactor with solid support or a flow-through reactor in which reagents flow continuously through the bed of solid-supported reactant. There is a fundamental problem with the synthesis on the planar arrays: solutions confined to individual spots cannot be stirred. The transport of the reagents, thus, is diffusion limited. On patterned paper, such as that described in US patent publication 20090298191 and other publications describing water-repelling paterns on paper, it is known that the excess of water confined by the hydrophobic pattern, over time, flows through the paper due to gravity. In this process, the solid support is exposed to a continuous flow of the solvent. There is limited use of this flow-through in patterned arrays to facilitate organic synthesis because such application would require the generation of patterns that retain organic solvents.

WO 2011103668 describes cancer specific peptides and arrays for screening the same. Synthesis is performed using standard SPOT. However, after synthesis the membrane is tested for short term cell adhesion only, not for any long-term culture or screening for phenotypic changes in cells.

The long-term culture of cells on or in cellulose or screening on or in synthetic materials has been described previously.

US 20110105360 "Paper-based cellular arrays" describes cells were cultured in 3D inside gels supported by paper.

CA 2662477 describes medical devices having a coating for promoting endothelial cell adhesion and US Patent No. 5,254,471 discloses a carrier for culturing cells made of ultra fine fibers.

WO 2009/126980 discloses cellulose-based hydrogel, which contains cellulose exhibiting an average degree of polymerization of 150-6200

WO 2012056110 describes cell culture material based on microbial cellulose.

One issue with these references is that they are not compatible for screening large numbers (i.e., >100) of chemically-modified 3D cellulose mesh to promote specific cellular responses (adhesion, growth, migration, differentiation, reprogramming). These features would be quite attractive in the area of development of bioactive material for cell-based studies, organic and cell culture, toxicology studies and development of therapeutic materials or diagnostic assays.

Other references describe screening for cell differentiation on arrays of peptide or synthetic materials for the purpose of discovery of new chemical compositions of materials that controls cell growth or differentiation. For example, US Patent No. 8,062,890 defines surfaces of self-assembled monolayers and stem cells; US patent publication 2010/0273259 describes substrates and methods for culturing stem cells; US patent publication 2005/0019747 discloses nanoliter-scale synthesis of arrayed biomaterials and screening thereof; US Patent No. 6,548,263 describes miniaturized cell array methods and apparatus for cell-based screening; US Patent No. 6,653,124 discloses array-based microenvironment for cell culturing, cell monitoring and drug-target validation; and WO 2007008609 describes methods and apparatus for cell culture array.

These examples of arrays are limited to cell-based screening on 2D surfaces or synthesis and screening on 2D surfaces; they do not describe synthesis and 3D culture of cells on the same support.

Other references describe screening for 3D hydrogels modified by peptide in which cells can grow for a prolong period of time. These include, for example, WO 2012009682, which describes elastic substrates and methods of use in cell manipulation and culture; and US patent publication 2010/0055733 which describes the manufacture and uses of reactive microcontact printing of biomolecules on soft hydrogels. However, these platforms do not allow for organic synthesis. All tested material must be synthesized separately and deposited onto 3D material. Ueda et al, Lab on a Chip, RSC, 1 January 2012 "Droplet Microarray: facile formation of arrays of microdroplets and hydrogel micropads for cell screening applications" discloses a super hydrophilic-super-hydrophobic patterned surface. WO 02/094846 A2 discloses apparatus and methods for the synthesis of arrays of molecules bound to a substrate. F Deiss et al. "Platform for High-ThroughputTesting of the effect of Soluble Compounds on 3D Cell Cultures", Analytical Chemistry, 85, No.17, 12 July 2013, pp 8085-8094 discloses a platform for high-throughput testing of the effect of soluble compounds on 3D cell cultures.

Thus, there exists a need to provide an improved paper-based array.

This background information is provided for the purpose of making known information believed by the applicant to be of possible relevance to the present invention. No admission is necessarily intended, nor should be construed, that any of the preceding information constitutes prior art against the present invention.

### SUMMARY

It is an object of the present application to address or ameliorate some of the disadvantages associated with previous paper-based arrays and methods for parallel organic synthesis and methods for screening for bioactive materials.

In accordance with one aspect of the present application there is provided a porous support for parallel organic synthesis according to the claims. The solvophobic area can be at least 1 mm in thickness.

In accordance with another aspect of the present application there is provided a method of synthesizing one or more compounds on a planar-based array according to the claims.

The method can further comprise the step after applying the one or more solvents of modifying the porous support to provide a support comprising the one or more compounds in the solvophilic area.

The solvophobic material can be a perfluorinated polymer such as Teflon.

In certain embodiments, one or more reagents are different from each other. This permits different reactions to take place in parallel.

In certain embodiments, there is provided in the step of applying one or more solvents in the solvophilic area, applying an excess of solvent thereby allowing the solvent to wick through the support to generate a flow of solvent. The flow of solvent is about 0.1 to 10 µL per minute.

In accordance with another aspect of the present application, there is provided a method of performing one or more biochemical assays on the porous support according to the claims.

The present application also provides a method of fabricating the support comprising the steps of: providing the porous support, applying a solvophobic material to a portion of the support surrounding a solvophilic area to establish a first solvophobic area which surrounds the solvophilic area; applying a second solvophobic material to a second portion of the support within the solvophilic area to form an outer solvophilic area between the first solvophobic and second solvophobic areas; applying a first solution within the solvophilic area; and applying a second solution within the solvophilic area.

In certain embodiments, the first solution is a protective aqueous solution and the second solution is a modifier solution. The protective aqueous solution can be sucrose, while the modifier solution can be a perfluorinated polymer such as Teflon AF. The pattern can be outlined by wax printing.

The second solvophobic area can have a thickness of about 0.5 mm.

In particular, the present application describes the design and production of a porous support, such as paper, that contains geometrically defined areas that confine or repel a selection of known organic solvents, with the exception of a few solvents (e.g., solvents with high content of fluorine and solvents infinitely miscible with perfluorinated solvents). In one specific embodiment, the present application provides a sheet of paper that contains periodic array of areas ("zones") that could be permeated by organic solvent ("solvophilic" or "hydrophilic") surrounded by borders that repel organic solvent ("solvophobic", "oleophobic" or "solvanophobic"). An excess of organic solvent can be added to each zone; solvents in different zones do not mix, thus allowing a user to perform multiple chemical reactions in parallel on one planar support. The present application also describes a method for generation and use of substrates that contain multiple regions of unmodified paper (termed "zones") surrounded by paper-patterned perfluorinated material (termed "barriers") that confine organic solvents. The barriers have a minimal thickness of about 1 millimeter and have the ability to repel organic solvent and withstand prolonged treatment with this organic solvent. The "zones" surrounded by the barrier can serve as a support for parallel, supported organic synthesis on paper or analysis procedures in which chemicals are applied in consecutive steps to the zones.

In certain embodiments, the synthesis of such arrays can be simplified and improved using a patterned support as described in the present application. For example, the present application provides paper-supported molecular arrays to be used to perform synthesis faster and more effectively than conventional arrays. In addition, the present application provides that local flow-through conditions can be achieved for tested organic solvents. Gravity-driven flow of the organic solvents through patterned area makes it possible to replicate conditions similar to a flow-through synthesis in a column.

In certain embodiments, the present application provides substrates that confine liquid to 96 zones on one planar support; the arrangement of zones is identical to a footprint of standard 12x8 = 96 well plate, and the shapes of the zones are square and the sizes are 3x3 mm. However, it is contemplated that areas of other sizes, shapes and arrangements can be generated.

In certain embodiments, the arrangement of the zones on the support and zone-to-zone distances that provide optimal long-term retention of liquids is provided. In embodiments having square shaped zones, the barrier thickness between the zones must be at least 1 mm. In the case of complex patterns when one barrier thickness cannot be defined (such as, for example, in the use of periodic arrays of circles), the barrier thickness separating two zones is at least 1 mm in thickness in the smallest dimension. The 1 mm dimension is based on studies of long-term stability of solvophobic pattern to treatment with organic solvent. Barriers of <1 mm have been shown to have high failure rate after prolonged exposure to organic solvents.

In certain embodiments, the present application describes a method for conversion of unpatented paper to a pattern that can confine organic solvents. No reports of mass-production and use of such patterns in the literature existed to date. The present application provides a well-defined pattern that for long-term retention of organic solvents.

The present application provides steps for automated production of the patterned paper using liquid-handling equipment designed for serial dispensing of liquids into specific areas. In accordance with one embodiment, there is provided a support frame made of a durable material such as aluminum or solvent resistant plastic (e.g., polypropylene). The frame makes it possible to interface the paper with robotic equipment designed to handle 96-well plates. Examples of mass-production procedure for a representative multi-channel dispenser are described herein.

The present application describes liquid handling conditions within a patterned array that facilitate and accelerate chemical synthesis on paper including local retention of reagents, local washing, local flow-through conditions, and replication of the reaction solution from one array to another array.

The present application provides multi-step synthesis of peptides on patterned support and improved yields of the coupling when compared to conventional synthesis on the non-patterned support. Further, the present application provides examples of format of assays that can be performed with a patterned array. The format of the assay is fundamentally different from assay performed on non-patterned paper. Spots of the solutions of the reagents can be deposited locally in each area of patterned paper to maximize exposure of the reagent for desired area of the paper while minimizing the use of the reagent overall.

The present application provides a solvent-repelling technique that improves chemical synthesis and defines cell adhesion areas. It also describes long-term culture and phenotypic responses (growth, migration, differentiation) of cells inside chemically modified 3D porous material.

The present application provides method for rapid screening of 3D biomaterial that promotes specific response in cells cultures in contact with (on or inside) that material.

The present application provides uses which include, but are not limited to, cell adhesion, spreading, cell division, migration in 2D and 3D, cell differentiation and dedifferentiation, change in resistance to drugs and other toxic agents, change in gene expression and protein concentrations in the cell, aggregation into 3D cellular structures with tissue-like morphology.

### BRIEF DESCRIPTION OF THE FIGURES

Figure 1 illustrates production of a Teflon patterned paper from wax-patterned paper. (A) Wax-printing (B) depositing sucrose solution (C) depositing Teflon solution (D) drying, and rinsing the protective solution.
Figure 2 illustrates automated production, including images of the workstation at different stages of the patterning process: deposition of sucrose solution (top); deposition of Teflon AF solution (bottom).
Figure 3 shows (A) a scheme of the holder used for automated patterning; (B) Image of the paper clamped in the holder; (C) Drying rack; (D) Examples of four different patterns made by automated patterning.
Figure 4 illustrates (A) a scheme describing long-term confinement or spreading of solvents; (B) comparison between wax-patterned paper, and Teflon-patterned paper to show differences in confining aqueous solutions up to evaporation; and (C-D) Teflon-patterned papers of different shapes confine various organic or aqueous solutions.

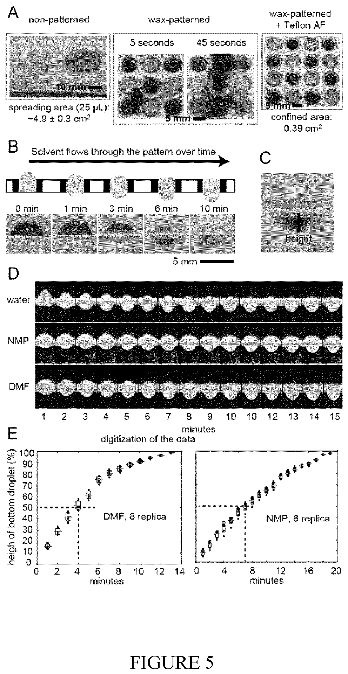

Figure 5 shows confinement of organic solvents in paper and flow-through. (A) Spotting 25 µL of DMF onto non-patterned paper yields area of 4.9 cm²; the same volume can be confined to a patterned area of 0.39 cm². (B) Solvents confined on paper flow through the pattern over time; (C) The process can be quantified by measuring the height of the droplet on either side of the paper (here bottom side); (D) Time lapse series describing flow of different solvents through the paper; (E) Digitization of the data from (D) from seven separate patterned area reveals that the rate of flow-through is reproducible for one type of solvent. Solvents of different viscosity (here DMF and NMP) exhibit different flow through rates.
Figure 6 shows different patterning geometries and their stability to long-term exposure to organic solvents. Patterns with <1 mm thick Teflon-containing barrier (or thicker wax-containing barrier) are less stable to the prolonged exposure to organic solvent.
Figure 7 illustrates activation of paper for peptide synthesis. Comparison of standard approach by immersion and approach by spotting. Yield of peptide synthesis, as quantified by the amount of Fmoc de-protected after attachment of amino acids, is significantly higher on Teflon-patterned arrays.
Figure 8 illustrates synthesis of decapeptide (Ala)₁₀ on Teflon-patterned paper and non-patterned paper and describes the yields of coupling at each step of synthesis.
Figure 9 illustrates other reactions on Teflon-patterned paper, including oxidation of the vicinal diols in peptides attached to paper and oxime bond coupling with the resulting aldehydes.
Figure 10 indicates adhesion of MDA-MB-231-GFP cells to known bioactive peptides synthesized on paper, using (A) fluorescent gel scanner and (B) confocal microscopy.
Figure 11 illustrates confocal imaging of MDA-MB-231-GFP cells on the peptide array presented in Figure 10.
Figure 12 illustrates long term cell growth on peptide-modified paper using (A) fluorescent gel scanner, (B) digitization of gel scanner images and (C) confocal fluorescent imaging.
Figure 13 illustrates representative confocal images of peptides synthesized on an array.
Figure 14 illustrates the adhesion of MDA-MB-231-GFP cells (A) using peptide arrays, (B) confocal imaging and (C) digitization of the number of cells per zone from fluorescent gel scanner images.
Figure 15 illustrates (A) an array imaged by a fluorescent gel scanner, (B) a list of sequences on the array and (C) confocal microscopy images.
Figure 16 illustrates representative confocal images of cells growing on peptide-modified paper.
Figure 17 shows LC-MS/UV analysis of a peptide (F). (A) Positive total ion count trace; (B) Negative total ion count trace; (C) UV trace; (D) - (E) MS spectra.
Figure 18 represents digitization of images: fluorescent gel scanner images (a) of replicates of array of 48 peptides (b) to extracted regions of interest (c) that are converted using a calibration curve (d) into a plot of cell numbers per peptide-zone (e).
Figure 19 illustrates confocal images of cells cultured on peptide-modified paper for seven days without (a) and with (b) anti-cancer drug.

### DETAILED DESCRIPTION

### Definitions

As used herein, an "organic solvent" is any commonly used organic liquid composed of carbon and hydrogen (including, for example, hydrocarbons such as hexane), oxygen (including, for example, alcohols, ethers, esters), nitrogen (including, for example, dimethylformamide, N-methyl pyrrolidone), chlorine (including, for example, dichloromethane), bromine, iodine or their combination.

As used herein, the volume of liquid retained by non-patterned paper represents an area S₁ of paper with thickness h and porosity p retains volume (V₁) defined as V₁ = S₁×h×p. Depositing excessive volume V₂ > V₁ leads to spreading of liquid and the increase of area S₁ to area S₂ to satisfy the relation V₂=S₂×h×p, and maintain the constant ratio of volume per surface area (Vᵢ/Sᵢ=const).

As used herein, "confinement of solvents" is defined as the ability of paper or other porous materials of area S surrounded by a solvophobic patterned border to retain a factor f of 3 to 100 larger volume (V_{conf}) of the solvent than this area S would normally retain. If the area is circular with radius r and the contact angle of solvent on solvophobic barrier is 90 degrees, V_{conf}=(2/3)πr³ and the volume retained by non-patterned paper of the same area is V=πhr, where h is the thickness of the paper. The ratio of two volumes yields f= 2r / 3h, which for a typical radius of 3 mm and thickness of 0.1 mm, yields a factor of 20 increase in volume that can be confined per surface area.

As used herein, the modification to the paper that allows confinement of organic solvents is referred to as "solvophobic modification" or "solvophobic patterning". The terms "solvophobic" and "oleophobic" describe the ability of the material to repel organic solvents; it is a general extension of the term "hydrophobic" (ability to repel water).

As used herein, "prolonged treatment with solvent" is defined as repeated exposure of the patterned material by complete immersion or by spotting of the selected areas with this solvent. The exposure lasts for at least 1 minute and is repeated at least 5 times. This condition constitutes a typical duration of exposure necessary for chemical reaction and for rinsing of the unreacted materials after the reaction.

As used herein, "mass-production" is defined as a reproducible automated, or semi-automated process that can be performed by a minimally trained person, who follows a short protocol, to generate many substrates per day, such as a hundred or more, for example. The process can be assisted and further accelerated by known liquid handling equipment such as a multichannel liquid dispenser or a programmable/robotic liquid dispenser.

As used herein, "supported organic synthesis" refers to any chemical transformation in which at least one of the reagents is immobilized via covalent or strong non-covalent bond to paper or other porous, planar insoluble support.

As used herein, "paper" refers to any porous, flat material made of cellulose. Materials with closely related properties, such as other hydrophilic, porous, insoluble polymers, either synthetic or natural, can also be used analogously.

Typically, any fluorochemical cellulose modifying agents known in the art for bulk paper modification can be combined with the "protection-deprotection" patterning method described herein (see steps (i)-(iii) below) to produce a patterned substrate that contain hydrophilic and oleophobic/solvophobic regions

General patterning steps include the following:
(i) The outlines of the patterns are designed on the computer and printed on paper using solid-wax printing (for example using Xerox Phaser DP 5600 solid ink printer). Dimension and arrangements of the patterns are such that satisfy requirements outlined above.
(ii) The areas to remain unmodified are protected by spotting an excess of solution of sucrose or other water-soluble hydrophilic material outlined in the "Detailed Description". Solution is retained by wax-pattern; it creates a convex aqueous droplet that protects that area from imbibition by fluorinated materials.
(iii) The remaining, unprotected areas are locally sprayed, spotted or coated by dispensing at a controlled flow rate the solution of Teflon AF in a suitable perfluorinated solvent (e.g., HFE 7100).
(iv) Evaporation of the perfluorinated solvent and rinsing the substrate with water to remove the sucrose yields a paper in which desired areas are hydrophilic and the rest are solvophobic/oleophobic.

As a further modification of the strategy described above, areas not protected by sucrose could be exposed to any known-in-the-art solution, material or treatment that modifies cellulose to make it oleophobic. The solution used for treatment should not be miscible or excessively reactive with aqueous protective solutions. An example of treatment that might be optimized by anyone trained in the art of organic synthesis is exposure of cellulose to the organic solvent that contains a reactive chemical that covalently links perfluorinated groups to cellulose. The reactions for introduction of such covalent bonds could be alkylation or esterification of the hydroxyl groups of the cellulose, their reaction with radical intermediates such as acrylate radicals or plasma-generated radicals, and multistep reactions. Many reactions that modify the paper with perfluorinated material and do not interfere with the strategy defined above could yield a paper modified with perfluorinated groups in a few well-defined areas (patterned paper).

The first step in patterning is to outline the features of the desired array using wax patterning or any technique that deposits well-resolved hydrophobic patterns through paper. Briefly, wax patterning can be performed as follows: (i) draw the desired pattern with the help of a computer, (ii) print it with a solid ink printer (e.g., Xerox Phaser), (iii) place the printed paper for 2-7 min in an oven at maintained at 120 °C-150 °C. Details and limitations of such patterning in outlining the hydrophobic borders can be found in Carrilho et al, "Understanding wax printing" Anal. Chem., 2009, 81 (16), pp 7091-7095.

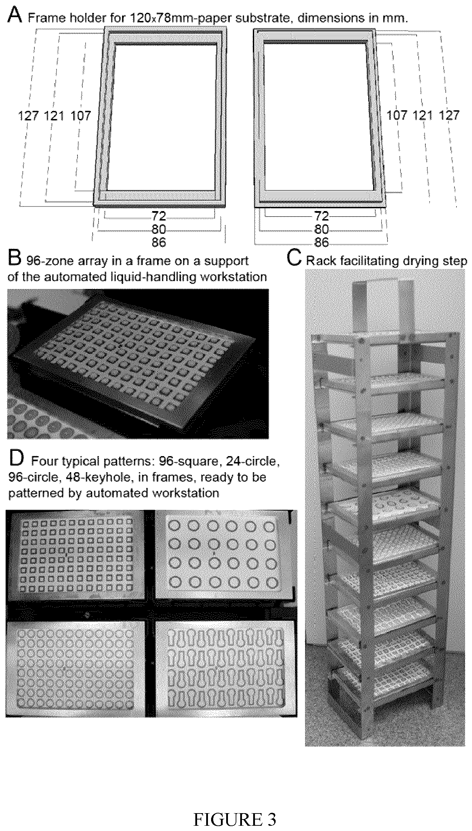

The paper is then cut to shape by a mechanical cutter, such as a die cutter or laser cutter to fit the paper into a metal holder. A typical holder is composed of two parts: a top and a bottom, both described in Figure 3, that grip the paper and support it throughout the Teflon patterning steps, as well as through following chemical modification steps and biochemical assays (as required). Two parts of the holder are designed with a tight fit; ideally, they are joined together by light pressing, held together by friction and can be assembled and disassembled reversibly. The holder described in Figure 3 has a footprint of standard multi-well plates and it was designed to maximize compatibility of Teflon-patterned paper with instruments manufactured to handle multi-well plates. One purpose of the holder is to prevent buckling of paper and to suspend the paper >2 mm above the surface and prevent any contact of paper with the surface as such contact would perturb patterned deposition of liquids. Other types of holders that clamp and suspend the paper, for example those that have a different footprint to fit different instruments, can be designed by small modifications of the design in Figure 3.

If necessary, additional fastening elements (such as screws, pins, springs, or clamps, for example) can be added to the holder. Such fastening might be beneficial for mass-production of frames. The "tight" fit between top and bottom requires high-precision manufacturing and could increase manufacturing costs on a mass production. On the other hand, a system with an insert screw would allow for more error margin in the production of top and bottom while maintaining the necessary fit/grip. A screw or other fastening may also ensure longer lifetime to the holder, as the frame dimensions and fit could be compromised by repeated opening and closing.

The second step of patterning is deposition of a hydrophilic protective solution, such as solution of 1 g/mL of sucrose in water, in the hydrophilic zones (or solvophilic). Any suitable liquid dispensing tool, such as multi-channel pipette, multichannel liquid handling robotics could be used at this step. The volume of the protective solution dispensed per zone depends on the design of the pattern and size of the zone. Large errors in volume can be tolerated in this step as long as liquid deposition yields a hemispherical (convex) drop. As examples, protection of a circular zone with 7-mm in diameter (Figure 3D, bottom-left) requires 30-60 µL of protective solution per zone, a square zone of 5x5 mm (Figure 3D, top-left) requires 15-40 µL per zone, a circular zone of 10.5-mm diameter (Figure 3D, top-right) required 60-100 µL per zone.

The sucrose solution described in the step above is chosen for its low cost, low evaporation rate and optimal viscosity. The combination of these factors allows for retaining a convex-shaped droplet of the liquid or amorphous "caramelized" material on the paper and protects it from any penetration by the Teflon AF solution (as described below). Even if a Teflon solution is deposited directly atop the droplet, the hydrophobic-hydrophilic repulsion and gravity-driven flow cause the Teflon solution to "roll off" the protected area. Protection by aqueous solutions that do not have convex shape appear to be less successful because such patterns can be coated by the Teflon film and such patterns require high precision in location and flow rate of the deposition of the Teflon solution. The patterning process can be optimized to work with ordinary water because the droplet of water has the ability to repel organic solvents; in addition, the water simply evaporates after patterning. Water droplets, however, have low viscosity and they flow through the pattern over time and lose convex shape. Water also evaporates faster than Teflon solution. Deposition of Teflon AF should happen within a short time interval after deposition of water drops. Ideally, evaporation of solvent used to dissolve Teflon AF should be done in conditions that prevent evaporation of water (low temperature, 100% humidity). The patterning process could also be optimized to work with the paper protected by the droplets of aqueous gels, such as agarose, that retain their shapes for prolonged time; such patterns, unlike sucrose or any aqueous solution patterns, would be stable to mechanical impacts and rotation. The pattern can also be turned sideways or upside down, immersed in coating solution, and dried in a geometry that maximizes patterning efficiency.

The third step in patterning is deposition of the solution of 20% Teflon AF (6.4mg/mL) (Dupont, grade 400S2-100-1; commercial solution at 32 mg/mL) solution in HFE-7100. The concentration of Teflon solution can be adjusted for the intended use of the paper array: a higher concentration of Teflon AF (20-40%; 6-13 mg/mL) may be needed if the paper is intended for the use in multi-step chemical synthesis with long and repeated exposures to organic solvent; lower concentrations (5-20%; 1.5-6 mg/mL) can be used for paper that will be exposed to organic solutions only briefly. Solutions of Telon AF with concentration below 15 mg/mL have low viscosity that allows these solutions to penetrate that paper at a sufficient rate during the patterning process. Concentrations higher than 20 mg/mL of Teflon AF form viscous solutions that cannot be handled easily by liquid dispensing system; they do not penetrate paper well and do not yield desired protection of paper from organic solvents.

The total volume of Teflon solution dispensed will depend on areas of the Teflon-covered surface ("background") in the specific pattern. Exemplary volumes required for the patterns described in Figure 3: the pattern with 96-circular zones has a background area of 57 cm² and requires 1.7 mL of a 6.4 mg/mL Teflon AF (Dupont, grade 400S2-100-1) solution in HFE-7100; a pattern with 96-square zones has a background area of 70 cm² and requires 2.2 mL of this solution; a pattern with 24-circle patterns has a background area of 73 cm² and 3.6 mL of solution of Teflon was used (for extended multi-steps synthesis).

After deposition, the solution of Teflon AF should be allowed to evaporate at room temperature. Drying can be accelerated by maintaining the arrays suspended (e.g. in a rack, Figure 3C) with at least 5 cm above and below the array. Drying can also be accelerated by exposing the array to the stream of hot air that does not perturb the shape of the aqueous droplets (e.g., heat gun, model MHT 750VT, Milwaukee Electric Tool; at a distance of >30 cm). The paper can be dried in an oven (e.g., 30 min at 60 °C). When optimizing other conditions, two factors should be considered: (1) high flow rate of air can cause disturbance in the shape of the drop and can lead to failure in patterning and deposition of Teflon in undesired areas; (2) high temperature during the drying process can bring the solution of sucrose to ebullition, which may cause "splashes" of protective solution in undesired regions of the array. Using the above examples and considerations, the drying conditions can be readily optimized by the person skilled in the art.

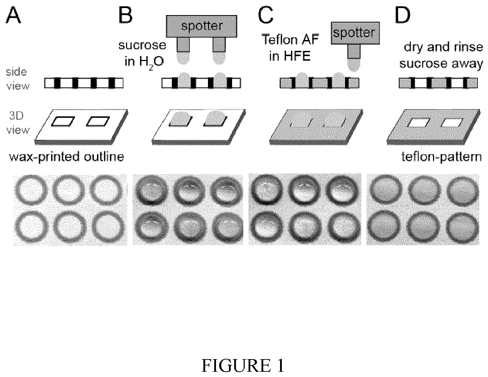

The drying step is accompanied by the visible changes in optical properties of the paper. Dried Teflon-patterned regions are non-transparent --they have bright-white color with high reflectance; by contrast, the hydrophilic, sucrose-protected regions on the same array appear semi-translucent (Figure 1D). Complete drying of the Teflon solution is important as regions that have not been dried completely yield imperfect patterning that is prone to failure during treatment with organic solvents.

As a final step, the array can be rinsed with water (e.g., under flow of water for 2-5 minutes, by immersion in a large container for at least 10 minutes, or any other suitable process that removes the protective solution). Exposure of the array to water during this step reveals any imperfections in patterning, which will appear as translucent regions in Teflon patterned areas. At this step, the arrays should be inspected for quality. Imperfect arrays can be returned to the patterning process (for example, repeated deposition of sucrose and Teflon is required). Multiple repeated depositions are possible, but not advisable.

The water-rinsed array is dried before the use, packaging or storage.

Automated patterning. Below are exemplary specific steps and experimental parameters input into the commercial robotic spotter (Precision XS workstation from BioTek, US). The program yields 4 patterned substrates (∼3.5 min/array) without any intervention from the user. The program detailed in the Program Report generates patterns of four different shapes and dispenses volumes of the protective solution and then Teflon solution described in [0081] and [0083] without any variations.

### PROGRAM REPORT

Program Name: TAFon4diffPatt
File Name: C:\ProgramData\BioTek\PrecisionPower\ProgramFiles\TAFon4diffPattern.PGM
Format: 8-Channel (using single channel pipettor)

### Comments

A= 96 circles
B=48 Keyhole shape
D=96 squares
E=24 circles

### Supply List

VESSELS in Station A columns 1 to 12 of type 96Circle, volume 0µl, reload after 1, reset after 1, No IDs
VESSELS in Station B columns 1 to 12 of type 96Circle, volume 0µl, reload after 1, reset after 1, No IDs
TIPS in Station C columns 1 to 12 of type LABCON200ROBOTIC, reload after 1, reset after 1, No IDs
VESSELS in Station D columns 1 to 12 of type 96Circle, volume 0µl, reload after 1, reset after 1, No IDs
VESSELS in Station E columns 1 to 6 of type 24-Circle, volume 0µl, reload after 1, reset after 1, No IDs

VESSELS in Station F columns 1 to 1 of type REAG_RES_UNIV, volume 50ml, reload after 1, reset after 1, No IDs

### Loop Information

Max Loop: 12
Prompt: Number of loops

### Program Commands

REMARK "Starting deposition on 96 circles"
LOOP Max times [Level 1]
TIPS from Sta. C column 1 (+0) using Rack for tips, Waste for residual
ASPIRATE 40ul from Sta. F column 1 (+0) using PIPAspSugar
DISPENSE 40ul into Sta. A column 1 (auto-incr) using PIPDispensePaper
LOOP OFF
LOOP 88 times [Level 1]
DISPENSE 15µl into Sta. A location A1 (auto-incr) by col using SingleDispTAF
LOOP OFF
LOOP 8 times [Level 1]
DISPENSE 25µl into Sta. A location A1 (auto-incr) by col using SingleTAFLeft
LOOP OFF
LOOP 8 times [Level 1]
DISPENSE 25µl into Sta. A location A12 (auto-incr) by col using SingleTAFRight
LOOP OFF
REMARK "Starting deposition on 96 squares"
LOOP Max times [Level 1]
TIPS from Sta. C column 1 (+0) using Rack for tips, Waste for residual
ASPIRATE 25µl from Sta. F column 1 (+0) using PIPAspSugar
DISPENSE 25µl into Sta. D column 1 (auto-incr) using PIPDispensePaper
LOOP OFF
LOOP 88 times [Level 1]
DISPENSE 20µl into Sta. D location A1 (auto-incr) by col using SingleDispTAF
LOOP OFF
LOOP 8 times [Level 1]
DISPENSE 25µl into Sta. D location A1 (auto-incr) by col using SingleTAFLeft
LOOP OFF
LOOP 8 times [Level 1]
DISPENSE 25µl into Sta. D location A12 (auto-incr) by col using SingleTAFRight
LOOP OFF
REMARK "Starting deposition on 24circles"
LOOP 6 times [Level 1]
TIPS from Sta. C column 2 (+0) using Rack for tips, Waste for residual ASPIRATE 75ul from Sta. F column 1 (+0) using PIPAspSugar
DISPENSE 75ul into Sta. E column 1 (auto-incr) using PIPDispensePaper
LOOP OFF
LOOP 48 times [Level 1]
DISPENSE 70µl into Sta. E location A1 (auto-incr) by col using SingleDispFor24
LOOP OFF
LOOP 8 times [Level 1]
DISPENSE 50µl into Sta. E location A1 (auto-incr) by col using SingDisp24left
LOOP OFF
LOOP 8 times [Level 1]
DISPENSE 50µl into Sta. E location A6 (auto-incr) by col using SingDisp24Right
LOOP OFF
REMARK "Starting deposition on 48 KH"
LOOP Max times [Level 1]
TIPS from Sta. C column 1 (+0) using Rack for tips, Waste for residual ASPIRATE 32µl from Sta. F column 1 (+0) using PIPAspSugar
DISPENSE 32µl into Sta. B column 1 (auto-incr) using PIPDispensePaper
LOOP OFF
LOOP 88 times [Level 1]
DISPENSE 13µl into Sta. B location A1 (auto-incr) by col using SingleDispTAFslw
LOOP OFF
LOOP 8 times [Level 1]
DISPENSE 20µl into Sta. B location A1 (auto-incr) by col using SingleTAFLeft
LOOP OFF
LOOP 8 times [Level 1]
DISPENSE 20µl into Sta. B location A12 (auto-incr) by col using SingleTAFRight
LOOP OFF
<End>

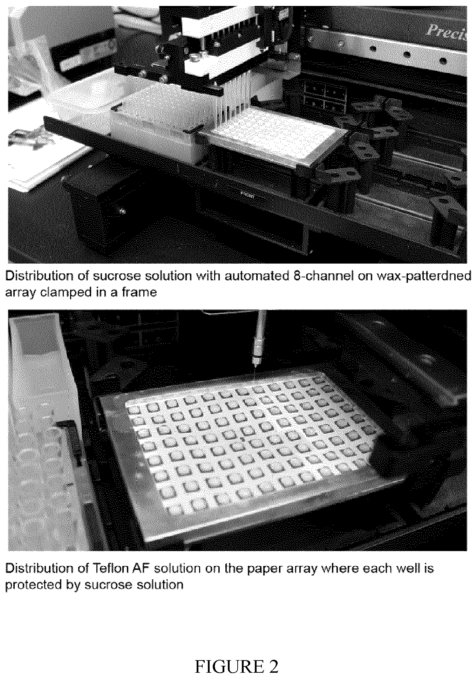

Automated Teflon-patterning using the holder is described in Figure 2. Another custom holder can be designed and input in this or similar liquid handling instrument. In the programming unit of the robot, the paper clamped by the holder is described as a micro plate vessel with the specific number of wells (96), dimensions in X, Y and Z, and maximum volume. It is important to define an accurate vertical limit displacement and height at which dispensing occurs to ensure that the tips of the liquid handler do not puncture the paper.

In particular embodiments, the liquid-handling system should be compatible with perfluorinated solvent (tubing should not be reactive to HFE solvent). Containment of the perfluorinated solution should prevent evaporation of the solvent. For example, in Precision XS, perfluorinated solution is dispensed from a sealed bottle. In this example, the concentration of the Teflon solution in the program was 6.4 mg/ml. Handling of solutions of higher concentrations needs to be adjusted appropriately to allow reproducible dispensing by the robot.

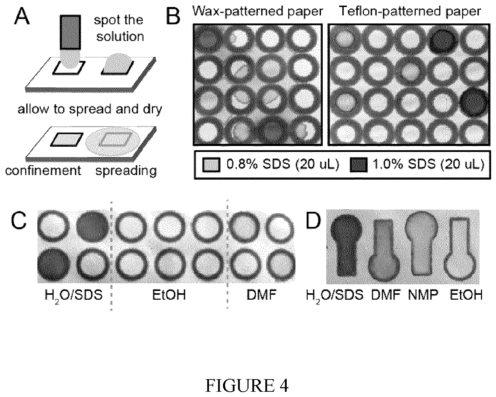

As shown in Figure 4, different solvents (organic, or aqueous with surfactant) that would not be confined by a simple wax-patterning can be confined in the well of a Teflon-patterned array for a long time (up to evaporation). Different geometries can be designed for various applications.

### EXAMPLES

Figure 5 illustrates one example which describes quantification of the rate of the flow of different solvents through the patterned area. Twenty microlitres of DMF, water or NMP were spotted on 5x5 mm hydrophilic area surrounded by Teflon pattern. The shape of the droplet of solvent every minute was monitored using a camera and the height of the droplet was quantified above and below the paper using custom image analysis script (MatLab). Good reproducibility in the rate of flow of liquids through the paper was observed. Different organic solvents have different flow-through rates. If the flow-through rate is approximated as the time required for 50% of the solvent to flow through the paper, this time was 2x faster for DMF than for NMP. Flow-through rate may be related to the viscosity of the solvent and optimal flow-through conditions may be designed and quantified using the method similar to that described in Figure 5. The flow can be accelerated, if necessary, by spinning the array in a centrifuge equipped with rotor for micro-well plates. Flow can also be accelerated by applying a suction of air under the array, for example a tube or array of tubes connected to vacuum underneath the array.

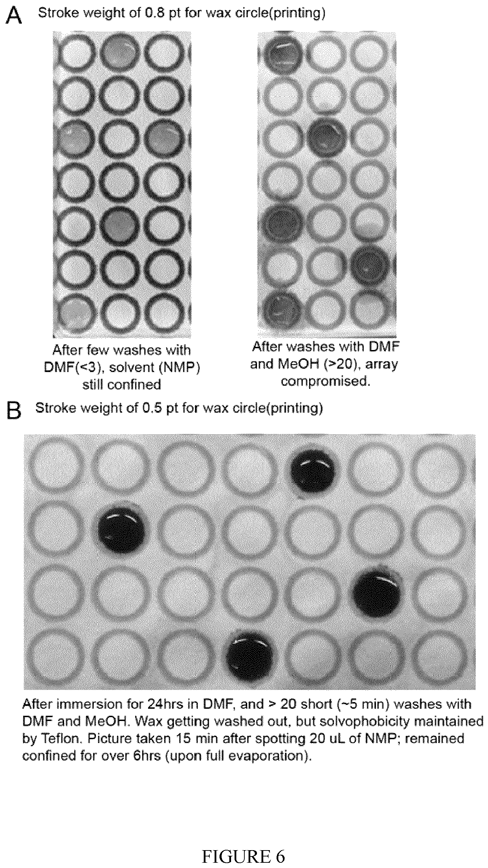

Figure 6 illustrates one example which describes assays to detect long-term stability to organic solvents and conditions in which pattern fails. The assay to detect failure of the pattern was the following: "short-term wash": the array was exposed to DMF for 3 cycles, 1 minute each; "long-term wash": the array was exposed for 20 cycles to DMF (1 minute each cycle) and/or 24 hour immersion in DMF. These conditions were selected to imitate the typical exposure of the arrays to the multi-step organic synthesis. The stroke thickness used to design the pattern during wax-patterning process influenced the stability of Teflon-patterned array; this stroke defined the thickness of the Teflon-containing barrier between the wells. A Teflon-patterned array with very small barriers of Teflon confined solvent only after "short term wash". After "long-term wash" this pattern failed (Figure 6A). The failure of this pattern was due to <1 mm thickness of the Teflon-protected area separating the wells. Reducing the wax stroke to a minimum thickness (e.g., 0.5 pt for 115 microns-thick paper) increased the thickness of the Teflon-containing barriers and makes patterns resistant to long-term wash (Figure 6B).

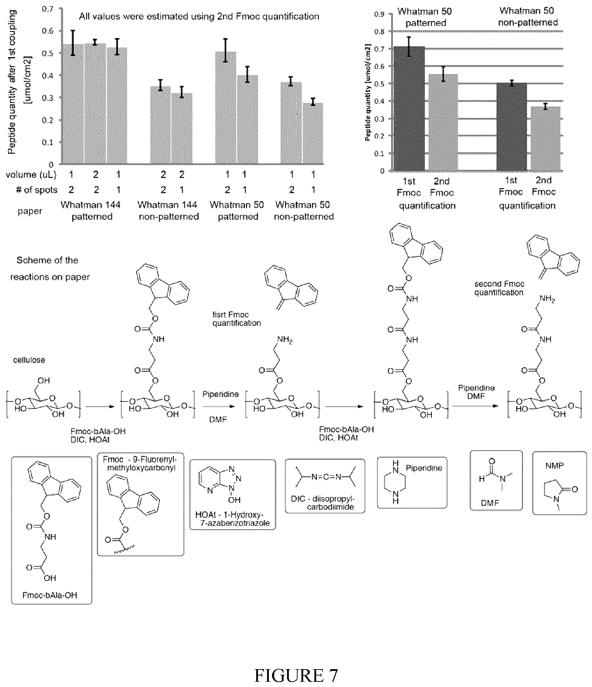

Figure 7 illustrates one example which demonstrated that patterned support has superior performance during coupling reactions using in standard peptide synthesis. Conditions and reagents are analogous to standard solid-phase synthesis on paper (SPOT). Coupling of amino acids and improved yield of synthesis were observed (Figure 7). Both patterned and non-patterned sheets of paper were exposed to standard amino acid coupling conditions and the amount of peptide synthesized per unit area was compared. To this end, the amount of Fmoc removed from each peptide attached to the solid phase was quantified. A significant increase in the amount of peptide synthesized on patterned arrays was observed when compared to non-patterned arrays (Figure 7).

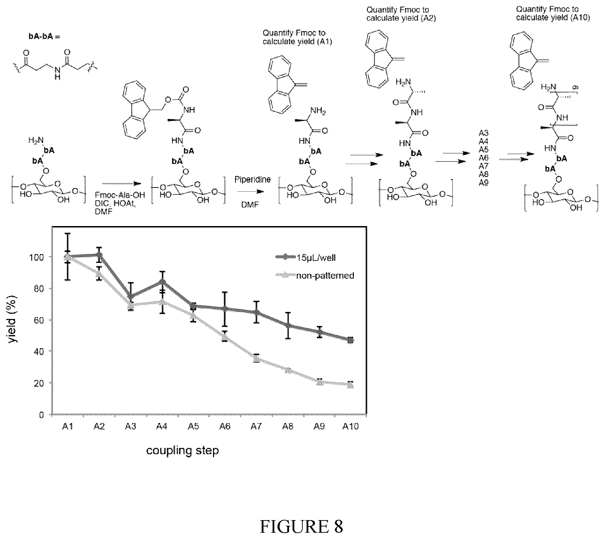

Figure 8 illustrated another example of superior performance of patterned paper in a 20-step synthesis of 10-mer peptide (Ala)₁₀. Comparison of the same syntheses on patterned support and non-patterned support demonstrates that the yields at most steps are significantly higher on patterned support. The yield of the final decapeptide (Ala)₁₀ is ∼20% on non-patterned support and ∼50% on the patterned support. Decreased yield incomplete peptide sequences at each reaction step. These incomplete sequences contaminate the main product and cause problems in purification and downstream assays. The anticipated purity of the final product made on patterned support is significantly higher than the purity of the same product generated on non-patterned support.

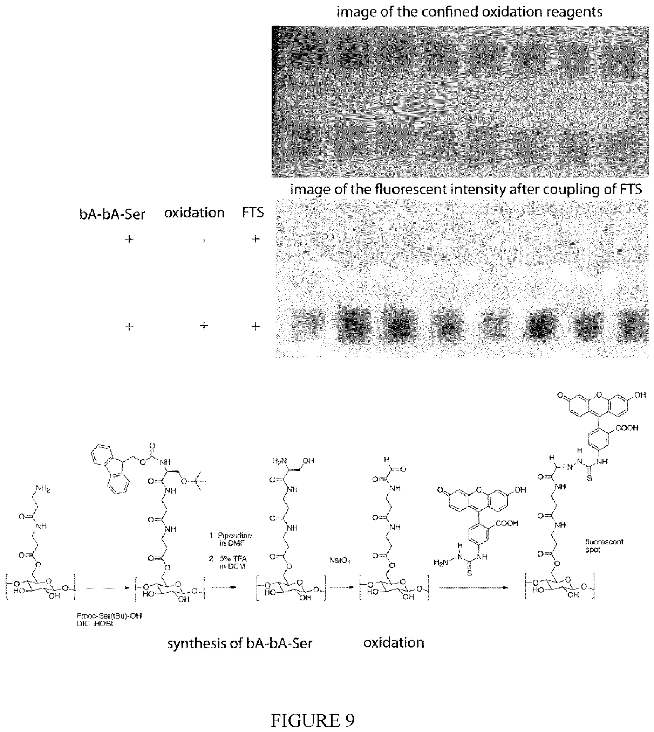

Figure 9 illustrates an example demonstrating the use of patterned array in oxidation of vicinal diols and oxime bond formation with the resulting aldehyde displayed on the solid support. Oxidation reagents (NaIO₄) were spotted only on selected areas and oxime was formed only in the areas that contained the oxidizing agent. Other chemical syntheses may be performed as known in the art of organic synthesis.

### EXAMPLE 2

It has been shown previously that paper can be used to generate "foldable" 3D tumor models to study 3D cultures of cells in vivo and in vitro (Derda et al., Proc. Natl. Acad. Sci. U. S. A. 2009, 106, 18457-18462), their migration (Derda et al., PLoS ONE 2011, 6, (5): e18940), and drug resistance (Deiss et al., Anal. Chem. 2013, 85, 8085-8094). The use of Teflon-patterned arrays allow for characterizing surface-immobilized peptides that can support cell adhesion, growth or differentiation. Bioactive peptides were synthesized and previously reported, which are known to support self-renewal of stem cells (Melkoumian et al., Nat. Biotechnol. 2010, 28, 606-610; Klim et al., Nat. Methods 2010, 7, 989-994; Derda et al., J. Am. Chem. Soc. 2010, 132, 1289-1295), and induce epithelial-mesenchymal transition (Li et al., Proc. Natl. Acad. Sci. U. S. A. 2011, 4377-4382).

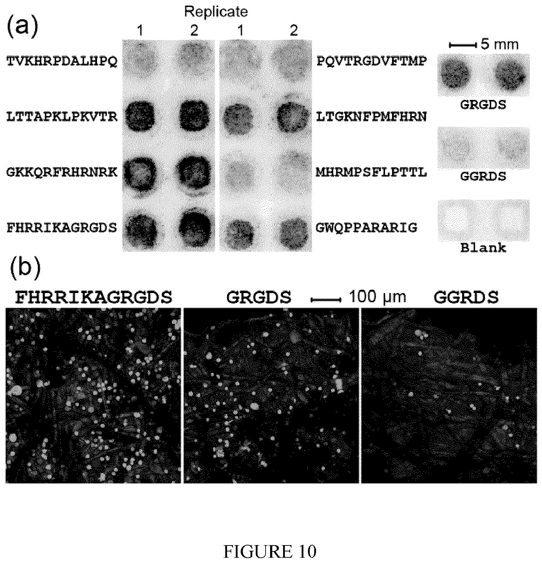

Figure 10 shows adhesion of MDA-MB-231-GFP cells to known bioactive peptides synthesized on paper. When immobilized on paper, 5 out of the 8 peptides synthesized supported adhesion of MDA-MB-231 breast carcinoma at levels similar to or higher than the positive control GRGDS. The other three peptides supported greater adhesion than the scrambled peptide, GGRDS. Unmodified cellulose and paper decorated with GGRDS exhibited minimal binding. Figure 10(a) shows imaging with a fluorescent gel scanner locates GFP fluorescence (dark areas). Figure 10(b) shows confirmatory results by confocal microscopy to validate binding to GRGDS (positive control) and no binding to GGRDS (negative control).

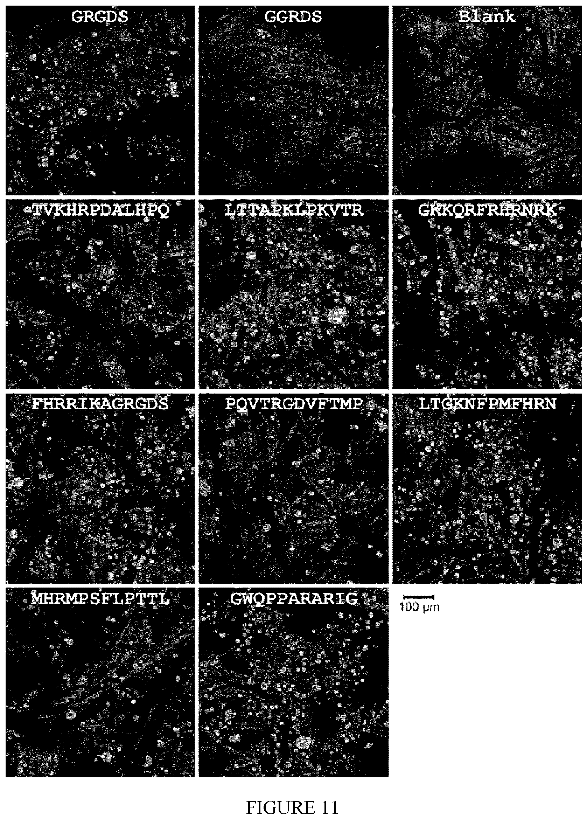

Figure 11 displays confocal micrographs of the MDA-MB-231-GFP cells on the peptide array presented in Figure 10. The images represent short term adhesion of cells after 3 hours of incubation on the array.

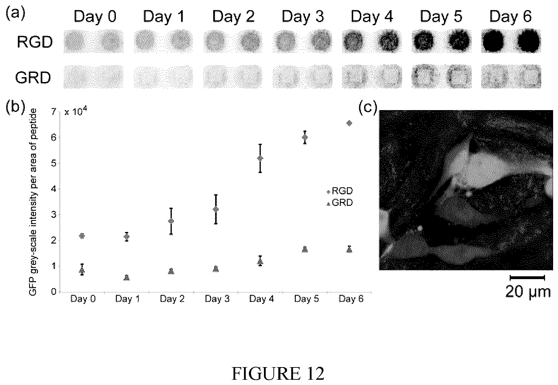

Figure 12 illustrates long term cell growth on peptide-modified paper. In Figure 12(a), fluorescent gel scanner images of MDA-MB-231 cells (transfected with GFP) on paper modified with GRGDS and GGRDS peptides are shown. The fluorescent intensity of cells on paper was tracked each day over the course of 6 days and recorded with the same grey-scale level. The increase in grey-scale indicates an increase in the number of cells.

Figure 12(b) shows the digitization of the grey-scale intensity of the gel scanner images. The grey-scale is proportional to the number of cells, and displays cell growth. Figure 12(c) shows a representative confocal fluorescent image of cells (green) adhering to fibers of the peptide-modified paper (blue). The image is taken on GRGDS-modified paper after 6 days of growth with cells. These results indicate that none of the components of the Teflon-patterned peptide array exhibited any toxicity to the cells.

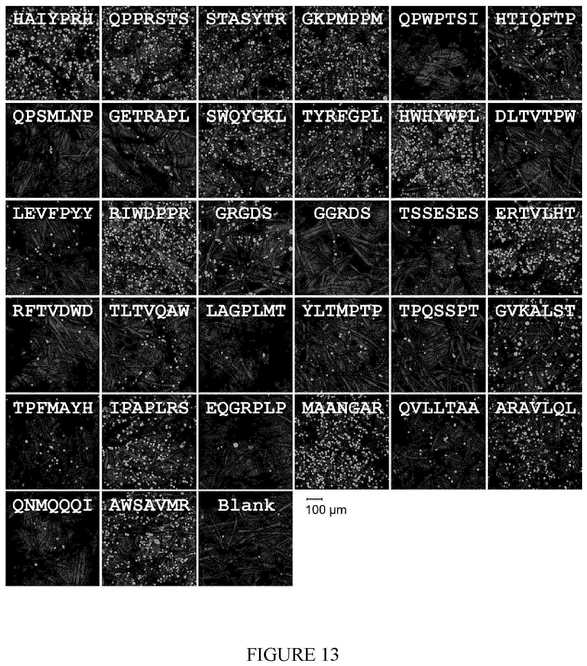

Figure 13 shows representative confocal images of peptides synthesized on an array. The images correspond to the peptide zone on the array scanned by a fluorescent gel scanner. Images represent short term adhesion of cells after 3 hours of incubation on the array. Over the long term, the cells on some peptide-modified surfaces spread along the fibers and resumed cell division.

Teflon-patterned arrays were then used to validate biological properties of 30 peptides identified *de novo* by phage display panning on MDA-MB-231 cells (Table 1).

| # | Peptide sequence | Length * | Replicates per array | Source | Estimated purity (LC-MS) |
|---|---|---|---|---|---|
| 1 | TVKHRPDALHPQ (SEQ ID NO.1) | 14-mer | 12 | ^{[2]} | 67 % |
| 2 | LTTAPKLPKVTR (SEQ ID NO.2) | 14-mer | 12 | ^{[2]} | 56 % |
| 3 | GKKQRFRHRNRK (SEQ ID NO.3) | 14-mer | 12 | ^{[3]} | low |
| 4 | FHRRIKAGRGDS (SEQ ID NO.4) | 14-mer | 12 | ^{[3]} | 22 % |
| 5 | PQVTRGDVFTMP (SEQ ID NO.5) | 14-mer | 12 | ^{[4]} | 18 % |
| 6 | LTGKNFPMFHRN (SEQ ID NO.6) | 14-mer | 12 | ^{[5]} | 31 % |
| 7 | MHRMPSFLPTTL (SEQ ID NO.7) | 14-mer | 12 | ^{[5]} | 59 % |
| 8 | GWQPPARARIG (SEQ ID NO.8) | 13-mer | 12 | ^{[3]} | 82 % |
| 9 | TYKYYPL (SEQ ID NO.9) | 9-mer | 12 | Panning A | 100% |
| 10 | HAIYPRH (SEQ ID NO.10) | 9-mer | 6 | Panning A | 10% |
| 11 | QPPRSTS (SEQ ID NO.11) | 9-mer | 6 | Panning A | 99% |
| 12 | STASYTR (SEQ ID NO.12) | 9-mer | 6 | Panning A | 100% |
| 13 | GKPMPPM (SEQ ID NO.13) | 9-mer | 6 | Panning A | 100% |
| 14 | QPWPTSI (SEQ ID NO.14) | 9-mer | 6 | Panning A | 86% |
| 15 | HTIQFTP (SEQ ID NO.15) | 9-mer | 6 | Panning A | 100% |
| 16 | QPSMLNP (SEQ ID NO.16) | 9-mer | 6 | Panning A | 88% |
| 17 | GETRAPL (SEQ ID NO.17) | 9-mer | 6 | Panning A | 100% |
| 18 | SWQYGKL (SEQ ID NO.18) | 9-mer | 6 | Panning A | 100% |
| 19 | TYRFGPL (SEQ ID NO.19) | 9-mer | 6 | Panning A | 54% |
| 20 | HWKYWPL (SEQ ID NO.20) | 9-mer | 6 | Panning A | 58% |
| 21 | DLTVTPW (SEQ ID NO.21) | 9-mer | 6 | Panning A | 86% |
| 22 | LEVFPYY (SEQ ID NO.22) | 9-mer | 6 | Panning A | 100% |
| 23 | RIWDPPR (SEQ ID NO.23) | 9-mer | 6 | Panning A | 80% |
| 24 | GRGDS (SEQ ID NO.24) | 7-mer | 6 | Panning A | 32% |
| 25 | GGRDS (SEQ ID NO.25) | 7-mer | 6 | Panning A | 26% |
| 26 | TSSESES (SEQ ID NO.26) | 9-mer | 6 | Panning B | 62% |
| 27 | ERTVLHT (SEQ ID NO.27) | 9-mer | 6 | Panning B | 90% |
| 28 | RFTVDWD (SEQ ID NO.28) | 9-mer | 6 | Panning B | 39% |
| 29 | TLTVQAW (SEQ ID NO.29) | 9-mer | 6 | Panning B | 34% |
| 30 | LAGPLMT (SEQ ID NO.30) | 9-mer | 6 | Panning B | 63% |
| 31 | YLTMPTP (SEQ ID NO.31) | 9-mer | 6 | Panning B | 70% |
| 32 | TPQSSPT (SEQ ID NO.32) | 9-mer | 6 | Panning B | 70% |
| 33 | GVKALST (SEQ ID NO.33) | 9-mer | 6 | Panning B | 61% |
| 34 | TPFMAYH (SEQ ID NO.34) | 9-mer | 6 | Panning B | 61% |
| 35 | IPAPLRS (SEQ ID NO.35) | 9-mer | 6 | Panning B | 95% |
| 36 | EQGRPLP (SEQ ID NO.36) | 9-mer | 6 | Panning B | 87% |
| 37 | MAANGAR (SEQ ID NO.37) | 9-mer | 6 | Panning B | 43% |
| 38 | QVLLTAA (SEQ ID NO.38) | 9-mer | 6 | Panning B | 53% |
| 39 | ARAVLQL (SEQ ID NO.39) | 9-mer | 6 | Panning B | 36% |
| 40 | QNMQQQI (SEQ ID NO.40) | 9-mer | 6 | Panning B | 65% |
| 41 | AWSAVMR (SEQ ID NO.41) | 9-mer | 6 | Panning B | 65% |
| 42 | NQLAGSG (SEQ ID NO.42) | 9-mer | 3 | Panning B | 49% |
| 43 | TYKFGTL (SEQ ID NO.43) | 9-mer | 3 | Panning B | 94% |
| 44 | HWHFGPL (SEQ ID NO.44) | 9-mer | 3 | Panning B | 75% |
| 45 | SWKFGPL (SEQ ID NO.45) | 9-mer | 3 | Panning B | 91% |
| 46 | TWKFSPL (SEQ ID NO.46) | 9-mer | 3 | Panning B | 99% |
| 47 | NVSGSHS (SEQ ID NO.47) | 9-mer | 3 | Panning B | 54% |
| 48 | SVLLPHR (SEQ ID NO.48) | 9-mer | 3 | Panning B | 92% |
| 49 | DAGQVSQ (SEQ ID NO.49) | 9-mer | 3 | Panning B | 86% |
| 50 | RLPSWHE (SEQ ID NO.50) | 9-mer | 3 | Panning B | 80% |
| 51 | AYPEPYV (SEQ ID NO.51) | 9-mer | 3 | Panning B | 86% |
| 52 | QPTHPTR (SEQ ID NO.52) | 9-mer | 3 | Panning B | 28% |
| 53 | APIWMHV (SEQ ID NO.53) | 9-mer | 3 | Panning B | 88% |
| 54 | ATWQLGT (SEQ ID NO.54) | 9-mer | 3 | Panning B | 21% |
| 55 | LHRQSSA (SEQ ID NO.55) | 9-mer | 3 | Panning B | 95% |
| 56 | ASWIPLP (SEQ ID NO.56) | 9-mer | 3 | Panning B | 99% |
| 57 | QQQYMAH (SEQ ID NO.57) | 9-mer | 3 | Panning B | 77% |
| 58 | STPATLI (SEQ ID NO.58) | 9-mer | 3 | Panning B | 79% |
| 59 | WSLSELH (SEQ ID NO.59) | 9-mer | 3 | Panning B | 93% |
| 60 | LPVRLDW (SEQ ID NO.60) | 9-mer | 3 | Panning B | 85% |
| 61 | QTWLEMG (SEQ ID NO.61) | 9-mer | 3 | Panning B | 33% |
| 62 | GPHNPTQ (SEQ ID NO.62) | 9-mer | 3 | Panning B | 81% |
| 63 | NDRPHMP (SEQ ID NO.63) | 9-mer | 3 | Panning B | 66% |
| 64 | VPNIVTQ (SEQ ID NO.64) | 9-mer | 3 | Panning B | 69% |
| 65 | AGSVIDT (SEQ ID NO.65) | 9-mer | 3 | Panning B | 95% |
| 66 | QAYHVSA (SEQ ID NO.66) | 9-mer | 3 | Panning B | 42% |
| 67 | SNMTRWH (SEQ ID NO.67) | 9-mer | 3 | Panning B | 12% |
| 68 | GRLDTGI (SEQ ID NO.68) | 9-mer | 3 | Panning B | 72% |
| 69 | ALQPQKH (SEQ ID NO.69) | 9-mer | 3 | Panning B | 64% |
| 70 | ASYSGTA (SEQ ID NO.70) | 9-mer | 3 | Panning A | 88% |
| 71 | ATLTHPP (SEQ ID NO.71) | 9-mer | 3 | Panning A | 59% |
| 72 | FPSTITP (SEQ ID NO.72) | 9-mer | 3 | Panning A | 95% |
| 73 | HPFEHFS (SEQ ID NO.73) | 9-mer | 3 | Panning A | 54% |
| 74 | IPTLPSS (SEQ ID NO.74) | 9-mer | 3 | Panning A | 65% |
| 75 | SILPYPY (SEQ ID NO.75) | 9-mer | 3 | Panning A | 57% |
| 76 | STFTKSP (SEQ ID NO.76) | 9-mer | 3 | Panning A | 81% |
| 77 | VTAHGGR (SEQ ID NO.77) | 9-mer | 3 | Panning A | 46% |
| 78 | HALGPSS (SEQ ID NO.78) | 9-mer | 3 | Panning A | 69% |
| 79 | HHSLTVT (SEQ ID NO.79) | 9-mer | 3 | Panning A | 38% |
| 80 | KAVHPLR (SEQ ID NO.80) | 9-mer | 3 | Panning A | 59% |
| 81 | SFVLPYY (SEQ ID NO.81) | 9-mer | 3 | Panning A | 79% |
| 82 | SHTAPLR (SEQ ID NO.82) | 9-mer | 3 | Panning A | 52% |
| 83 | SPTQPKS (SEQ ID NO.83) | 9-mer | 3 | Panning A | 59% |
| 84 | SSLVRTA (SEQ ID NO.84) | 9-mer | 3 | Panning A | 72% |
| 85 | TARYPSW (SEQ ID NO.85) | 9-mer | 3 | Panning A | 32% |
| 86 | TFAKSAY (SEQ ID NO.86) | 9-mer | 3 | Panning A | 71% |
| 87 | TPPTMDH (SEQ ID NO.87) | 9-mer | 3 | Panning A | 57% |
| 88 | VIPHVLS (SEQ ID NO.88) | 9-mer | 3 | Panning A | 26% |
| 89 | WTITKHP (SEQ ID NO.89) | 9-mer | 3 | Panning A | 78% |
| 90 | YAGPYQH (SEQ ID NO.90) | 9-mer | 3 | Panning A | 74% |
| 91 | YKHPPQH (SEQ ID NO.91) | 9-mer | 3 | Panning A | 35% |
| 92 | AMSSRSL (SEQ ID NO.92) | 9-mer | 3 | Panning A | 70% |
| 93 | MDAHHAL (SEQ ID NO.93) | 9-mer | 3 | Panning A | 71% |
| 94 | NHWASPR (SEQ ID NO.94) | 9-mer | 3 | Panning A | 33% |
| 95 | STIHGST (SEQ ID NO.95) | 9-mer | 3 | Panning A | 40% |
| 96 | DSHTPQR (SEQ ID NO.96) | 9-mer | 3 | Panning A | 73% |
| 97 | NPPSRHP (SEQ ID NO.97) | 9-mer | 3 | Panning A | 41% |
| 98 | NQLPLHA (SEQ ID NO.98) | 9-mer | 3 | Panning A | 27% |
| 99 | SHALRTV (SEQ ID NO.99) | 9-mer | 3 | Panning A | 44% |
| 100 | SLPFQLT (SEQ ID NO.100) | 9-mer | 3 | Panning A | 70% |
| 101 | SPQMTLS (SEQ ID NO.101) | 9-mer | 3 | Panning A | 49% |
| Legend: | | | | | |
| *length including the two first βAla | | | | | |
| | | | | | |
| Panning A: panning of PhD library (lot 0061101) against GFP-MDA-MB-231 cells | | | | | |
| Panning B: panning of PhD library (lot 0081212) against GFP-MDA-MB-231 cells | | | | | |
| | | | | | |
| References: | | | | | |
| [2] | R. Derda, S. Musah, B. P. Orner, J. R. Klim, L. Y. Li, L. L. Kiessling, J. Am. Chem. Soc. 2010, 132, 1289-1295. | | | | |
| [3] | J. R. Klim, L. Y. Li, P. J. Wrighton, M. S. Piekarczyk, L. L. Kiessling, Nat. Methods 2010, 7, 989-994. | | | | |
| [4] | Z. Melkoumian, et al., Nat. Biotechnol. 2010, 28, 606-610; D. H. M. Hervy, M. Denise, M. Pecheul, C. Walerack (Corning Incorporated), WO2012150475 A1, 2011. | | | | |
| [5] | L. Y. Li, J. R. Klim, R. Derda, A. H. Courtney, L. L. Kiessling, Proc. Natl. Acad. Sci. U. S. A. 2011, 4377-4382. | | | | |

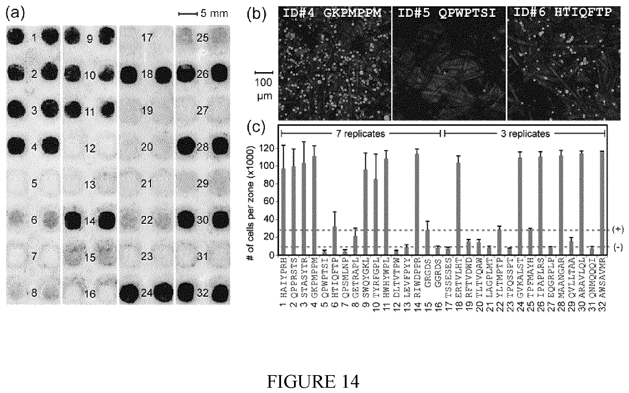

In Figure 14(a), a fluorescent gel scanner was used to validate adhesion of MDA-MB-231-GFP cells to phage-derived peptides on peptide-arrays. Figure 14(b) shows representative confocal images of peptide-modified paper exhibiting high, none and moderate cell adhesion (More examples of confocal images: Figures 13, 15, and 16). Figure 14(c) illustrates digitization of the number of cells per zone from fluorescent gel scanner images of the 32 peptides. The (+) and (-) lines represent the intensity level of the positive and negative controls. Overall, 14 out of 30 peptides supported cell adhesion at levels higher than the integrin-binding peptide GRGDS (Figure 14c); four peptides supported adhesion at similar levels, and 10 exhibited no adhesion.

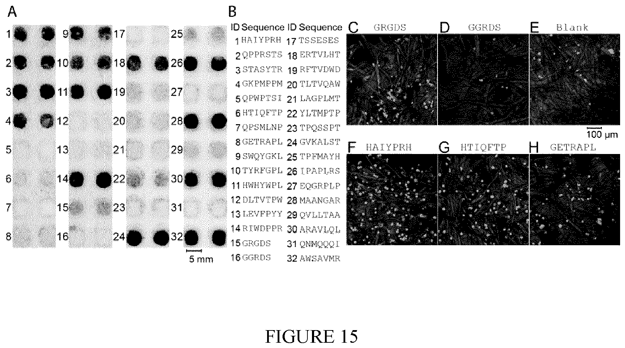

In Figure 15A, the array was imaged by a fluorescent gel scanner to identify the presence of cells (dark areas) on the array after 48 hours. Figure 15B lists the sequences that correspond to the fluorescent gel scanner image of the array. Cells (green) were observed on paper fibres (blue) by confocal microscopy to validate survivability of cells in paper. We observe attachment of cells and their spreading along the fibres of the paper support. Confocal images represent GRGDS (positive control) (Figure 15C), GGRDS (Figure 15D), blank paper (negative controls) (Figure 15E), and peptides with higher (Figure 15F), similar (Figure 15G) and lower (Figure 15H) binding to cells than GRGDS.

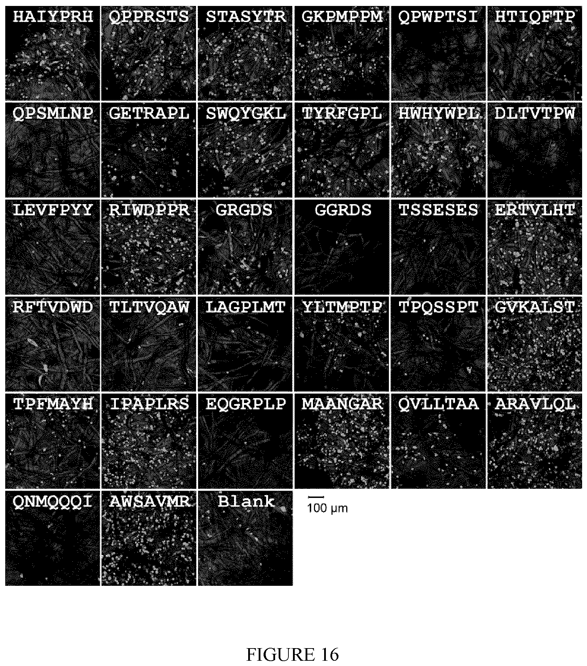

Figure 16 shows representative confocal images of cells growing inside peptide-modified paper after 48 hours of culture. The images correspond to the peptide zone on the array scanned by a fluorescent gel scanner.

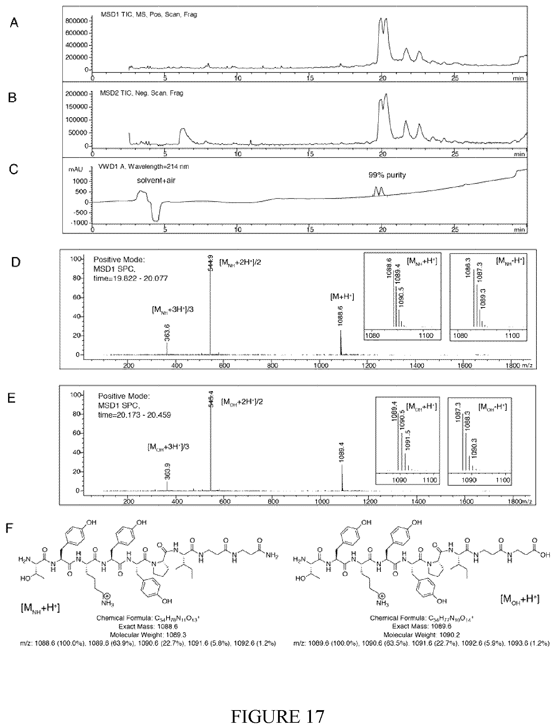

Figure 17 illustrates an example of LC-MS/UV analysis for peptide with >99% purity. Figure 17A shows a positive total ion count (TIC) trace; Figure 17B shows a negative TIC trace; Figure 17C shows a UV trace; and Figures 17D-E show MS spectra of peaks at the indicated elution time. Cleavage of peptides from the paper by NH3 gas was noted to yield a mixture of acid and amide functionalities on the C-terminus. These two peptides appear as doubles separated by <1 minute on LC trace.

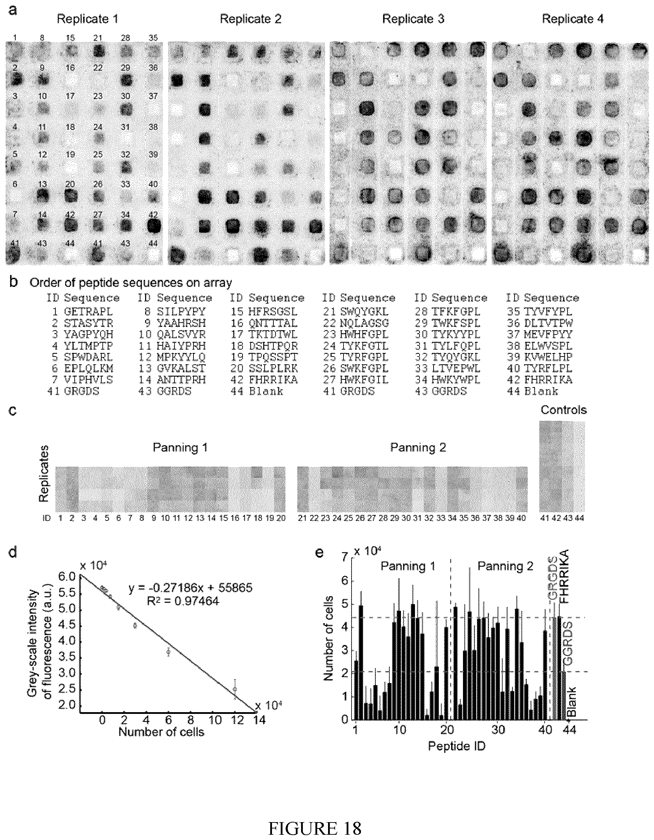

Figure 18 illustrates a typical cell-binding experiment on a half array (40 peptides identified from two panning experiments, and 8 controls). Figure 18a displays the fluorescent gel scanner images of four replicates, after exposure for 3 hours to MDA-MB-231-GFP cells. The list of peptide sequences on the array is given in Figure 18b. Figure 18c consists of the zones of interest for each peptide extracted from Figure 18a, and organized by peptides. The average intensity of fluorescence for each peptide zone is then converted into a number of cells per zone through a calibration curve previously established for these cells (Figure 18d). Figure 18e is the resulting plot with standard deviation over the four replicates.

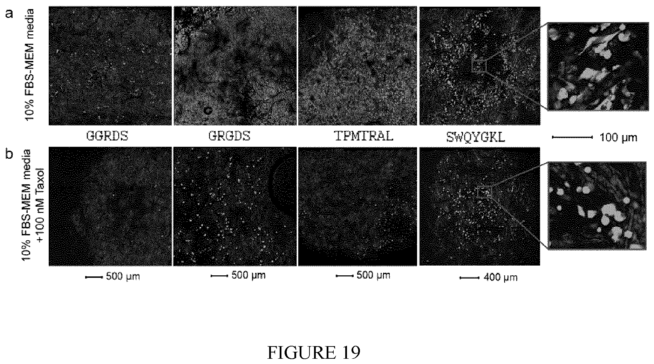

Figure 19 illustrates changes such as drug-resistance and morphology observed when a peptide-modified array supported culture of MCF-7 breast cancer cells for seven days and was exposed to the anti-cancer drug paclitaxel (Taxol) for the last 4 days. Figure 19a presents confocal images of calcein-stained cells after culture in media without taxol for four peptides (including two controls) and Figure 19b correspond to cells cultured in media with Taxol. The fourth peptide exhibits a population of drug-resistant cells. The last micrograph of each row is a magnification for cells cultured on a peptide that induced morphological changes: isolated, rounded cells when exposed to Taxol, instead of spread cells forming aggregate when cultured without drugs.

These experiments confirmed that Teflon-patterned paper is an effective platform for synthesis and cell-based screening of a large number of peptides. Paper is a versatile support for applications such as analytical devices or low-cost diagnostics (for recent reviews see Yetisen et al., Lab Chip 2013, 13, 2210-2251; Hossain et al., Anal. Chem. 2009, 81, 9055-9064). Parallel synthesis capability and generation of patterns resistant to organic solvents and surfactants should also be beneficial in these areas. Patterning of low-cost paper makes this technology conveniently available; however, future advances in materials production (lithography, 3D-printing, weaving, etc.; Tian et al., Chem. Soc. Rev. 2013, 42, 5184-5209) could yield similar low-cost, self-supported, patterned, porous sheets suitable for organic synthesis and bioassays.

## Claims

1. A porous support for parallel organic synthesis which is a paper-based array comprising:
a sheet of paper that contains a periodic array of areas;
a solvophilic area for spotting and permeating an organic solvent comprising a reagent for synthesizing an organic compound; and
a solvophobic area that surrounds the solvophilic area wherein the solvophobic area comprises a perfluorinated material such as perfluorinated polymer that repels the organic solvent.

2. The porous support of claim 1, wherein the perfluorinated polymer is Teflon.

3. A method of synthesizing one or more compounds on a planar-based array comprising
providing a porous support wherein the porous support is paper;
applying a solvophobic material to a portion of the support surrounding a solvophilic area to establish a solvophobic area which surrounds the solvophilic area;
wherein the solvophobic material is a perfluorinated material;
applying one or more solvents comprising one or more reagents in the solvophilic area, thereby confining the one or more solvents in the solvophilic area; and
synthesizing the one or more compounds from the one or more solvents.

4. The method of claim 3, further comprising the step after applying the one or more solvents of modifying the porous support to provide a support comprising the one or more compounds in the solvophilic area.

5. The method of any one of claims 3 to 4, wherein the solvophobic material is a perfluorinated polymer such as Teflon.

6. The method of any one of claims 3 to 5, wherein the one or more reagents are different from each other.

7. The method of any one of claims 3 to 6, wherein in the step of applying one or more solvents in the solvophilic area, an excess of solvent is applied thereby allowing the solvent to wick through the support to generate a flow of solvent, preferably at a flow of about 0.1 to 10 µL per minute.

8. The method of claim 3 or 4 wherein the compound synthesized is a peptide.

9. A method of performing one or more biochemical assays on the porous support of any one of claims 1 or 2, comprising
applying one or more reagents in the solvophilic area, thereby confining the reagents within the solvophilic area; and
performing the one or more biochemical assays.

10. A method of solvophobic patterning of paper or any hydrophilic, porous, insoluble polymers, either synthetic or natural comprising the steps of:-
outlining the features of the desired array using a technique that deposits well-resolved hydrophobic patterns through paper such as wax patterning;
deposition of a hydrophilic protective solution in the hydrophilic zones;
deposition of the solution of Teflon AF with concentration below 15 mg/mL to produce solvophobic zones which surround the hydrophilic zones;
drying the solution of Teflon AF;
rinsing with water or any suitable process that removes the hydrophilic protective solution.

11. Use of the porous support of any one of claims 1 or 2 , to identify a bioactive synthetic molecule, preferably wherein the bioactive molecule is a peptide or chemically-modified peptide.

12. The use of claim 11, wherein the bioactive molecule binds to a receptor biomolecule, where the receptor is a protein, carbohydrate, nucleic acid or other biological structure.

13. The use of claim 11, wherein the bioactive molecule binds to a receptor on a surface of cells and change biochemical or physiological properties of the cell, preferably wherein the properties include adhesion, spreading, migration, cell division, differentiation, change in gene expression, or cell death.

## Patentansprüche

1. Poröser Träger zur parallelen organischen Synthese, bei dem es sich um eine Anordnung auf Papierbasis handelt, umfassend:
ein Blatt Papier, das eine periodische Anordnung von Bereichen enthält;
einen solvophilen Bereich zum Auftropfen und Durchdringen eines organischen Lösungsmittels, das ein Reagenz zum Synthetisieren einer organischen Verbindung umfasst; und
einen solvophoben Bereich, der den solvophilen Bereich umgibt, wobei der solvophobe Bereich ein perfluoriertes Material, wie ein perfluoriertes Polymer, umfasst, das das organische Lösungsmittel abstößt.

2. Poröser Träger nach Anspruch 1, wobei das perfluorierte Polymer Teflon ist.

3. Verfahren zum Synthetisieren einer oder mehrerer Verbindungen auf einer planaren Anordnung, umfassend
Bereitstellen eines porösen Trägers, wobei der poröse Träger Papier ist;
Aufbringen eines solvophoben Materials auf einen Abschnitt des Trägers, der einen solvophilen Bereich umgibt, um einen solvophoben Bereich zu schaffen, der den solvophilen Bereich umgibt;
wobei das solvophobe Material ein perfluoriertes Material ist;
Aufbringen eines oder mehrerer Lösungsmittel, umfassend ein oder mehrere Reagenzien, im solvophilen Bereich, wodurch das eine oder die mehreren Lösungsmittel auf den solvophilen Bereich beschränkt werden; und
Synthetisieren der einen oder mehreren Verbindungen aus dem einen oder den mehreren Lösungsmitteln.

4. Verfahren nach Anspruch 3, das ferner nach dem Aufbringen des einen oder der mehreren Lösungsmittel den Schritt Modifizieren des porösen Trägers umfasst, um einen Träger bereitzustellen, der die eine oder die mehreren Verbindungen im solvophilen Bereich umfasst.

5. Verfahren nach einem der Ansprüche 3 bis 4, wobei das solvophobe Material ein perfluoriertes Polymer wie Teflon ist.

6. Verfahren nach einem der Ansprüche 3 bis 5, wobei das eine oder die mehreren Reagenzien voneinander verschieden sind.

7. Verfahren nach einem der Ansprüche 3 bis 6, wobei im Schritt Aufbringen eines oder mehrerer Lösungsmittel im solvophilen Bereich ein Überschuss an Lösungsmittel aufgebracht wird, wodurch das Lösungsmittel unter Erzeugen eines Lösungsmittelflusses, vorzugsweise mit einem Fluss von etwa 0,1 bis 10 µl pro Minute, durch den Träger gesogen werden kann.

8. Verfahren nach Anspruch 3 oder 4, wobei die synthetisierte Verbindung ein Peptid ist.

9. Verfahren zum Durchführen eines oder mehrerer biochemischer Assays auf dem porösen Träger nach einem der Ansprüche 1 oder 2, umfassend
Aufbringen eines oder mehrerer Reagenzien im solvophilen Bereich, wodurch die Reagenzien auf den solvophilen Bereich beschränkt werden; und
Durchführen des einen oder der mehreren biochemischen Assays.

10. Verfahren zur solvophoben Strukturierung von Papier oder beliebigen hydrophilen, porösen, unlöslichen, entweder synthetischen oder natürlichen Polymeren, umfassend die Schritte:
Umgrenzen der Merkmale der gewünschten Anordnung unter Verwendung einer Technik, die gut aufgelöste hydrophobe Muster durch Papier hindurch aufbringt, wie Wachsdruck;
Aufbringen einer hydrophilen Schutzlösung in den hydrophilen Zonen;
Aufbringen der Lösung von Teflon AF in einer Konzentration unter 15 mg/ml zur Herstellung solvophober Zonen, die die hydrophilen Zonen umgeben;
Trocknen der Lösung von Teflon AF;
Spülen mit Wasser oder beliebiges geeignetes Verfahren, das die hydrophile Schutzlösung entfernt.

11. Verwendung des porösen Trägers nach einem der Ansprüche 1 oder 2 zum Identifizieren eines bioaktiven synthetischen Moleküls, wobei bevorzugt das bioaktive Molekül ein Peptid oder ein chemisch modifiziertes Peptid ist.

12. Verwendung nach Anspruch 11, wobei das bioaktive Molekül an ein Rezeptorbiomolekül bindet, wobei der Rezeptor ein Protein, ein Kohlenhydrat, eine Nukleinsäure oder eine andere biologische Struktur ist.

13. Verwendung nach Anspruch 11, wobei das bioaktive Molekül an einen Rezeptor auf einer Oberfläche von Zellen bindet und die biochemischen oder physiologischen Eigenschaften der Zelle verändert, wobei bevorzugt zu den Eigenschaften Adhäsion, Ausbreitung, Migration, Zellteilung, Differenzierung, Veränderung der Genexpression oder Zelltod gehören.

## Revendications

1. Support poreux pour la synthèse organique parallèle qui est un réseau à base de papier comprenant :
une feuille de papier qui contient un réseau périodique de domaines :
un domaine solvophile pour le dépôt et la perméation d'un solvant organique comprenant un réactif pour la synthèse d'un composé organique ; et
un domaine solvophobe qui entoure le domaine solvophile, le domaine solvophobe comprenant un matériau perfluoré tel qu'un polymère perfluoré qui repousse le solvant organique.

2. Support poreux selon la revendication 1, le polymère perfluoré étant le Téflon.

3. Procédé de synthèse d'un ou plusieurs composés sur un réseau à base plane comprenant
la mise à disposition d'un support poreux, le support poreux étant du papier ;
l'application d'un matériau solvophobe à une partie du support entourant un domaine solvophile pour établir un domaine solvophobe qui entoure le domaine solvophile ;
le matériau solvophobe étant un matériau perfluoré ;
l'application d'un ou plusieurs solvants comprenant un ou plusieurs réactifs dans le domaine solvophile, confinant ainsi le ou les solvants dans le domaine solvophile ; et
la synthèse du ou des composés de l'un ou des solvants.

4. Procédé selon la revendication 3, comprenant en outre l'étape, après l'application du ou des solvants, de modification du support poreux pour fournir un support comprenant le ou les composés dans le domaine solvophile.

5. Procédé selon l'une quelconque des revendications 3 et 4, le matériau solvophobe étant un polymère perfluoré tel que le Téflon.

6. Procédé selon l'une quelconque des revendications 3 à 5, le ou les réactifs étant différents les uns des autres.

7. Procédé selon l'une quelconque des revendications 3 à 6, dans lequel dans l'étape d'application d'un ou plusieurs solvants dans le domaine solvophile, un excès de solvant est appliqué permettant ainsi au solvant de passer à travers le support pour générer un flux de solvant, préférablement à raison d'un flux d'environ 0,1 à 10 µL par minute.

8. Procédé selon la revendication 3 ou 4, le composé synthétisé étant un peptide.

9. Procédé de réalisation d'une ou plusieurs analyses biochimiques sur le support poreux selon l'une quelconque des revendications 1 et 2, comprenant l'application d'un ou plusieurs réactifs dans le domaine solvophile, confinant ainsi les réactifs à l'intérieur du domaine solvophile ; et
la réalisation de l'analyse ou des analyses biochimiques.

10. Procédé de structuration solvophobe de papier ou de quelconques polymères hydrophiles, poreux, insolubles, soit synthétiques, soit naturels comprenant les étapes de :
définition des caractéristiques du réseau souhaité à l'aide d'une technique qui dépose des motifs hydrophobes bien résolus à travers du papier telle que la structuration à la cire ;
dépôt d'une solution protectrice hydrophile dans les zones hydrophiles ;
dépôt de la solution de Téflon AF avec une concentration inférieure à 15 mg/mL pour produire des zones solvophobes qui entourent les zones hydrophiles ;
séchage de la solution de Téflon AF ;
rinçage avec de l'eau ou un quelconque procédé approprié qui élimine la solution protectrice hydrophile.

11. Utilisation du support poreux selon l'une quelconque des revendications 1 et 2, pour identifier une molécule synthétique bioactive, préférablement la molécule bioactive étant un peptide ou un peptide chimiquement modifié.

12. Utilisation selon la revendication 11, la molécule bioactive se liant à une biomolécule de type récepteur, le récepteur étant une protéine, un glucide, un acide nucléique ou une autre structure biologique.

13. Utilisation selon la revendication 11, la molécule bioactive se liant à un récepteur sur une surface de cellules et changeant des propriétés biochimiques ou physiologiques de la cellule, préférablement les propriétés comprenant l'adhérence, la propagation, la migration, la division cellulaire, la différenciation, un changement dans l'expression génique, ou la mort cellulaire.


### SEQUENCE LISTING

<110> The Governors of the University of Alberta
<120> PARALLEL ORGANIC SYNTHESIS ON PATTERNED PAPER USING A SOLVENT-REPELLING MATERIAL
<130> PPCT22382
<150> US 61/970,998
   <151> 2014-03-27
<150> US 61/847,647
   <151> 2013-07-18
<160> 101
<170> PatentIn version 3.5
<210> 1
   <211> 12
   <212> PRT
   <213> Artificial Sequence
<220>
   <223> peptide identified by phage display panning on MDA-MB-231 cells
<400> 1
   Thr Val Lys His Arg Pro Asp Ala Leu His Pro Gln
   1               5                   10
<210> 2
   <211> 12
   <212> PRT
   <213> Artificial Sequence
<220>
   <223> peptide identified by phage display panning on MDA-MB-231 cells
<400> 2
   Leu Thr Thr Ala Pro Lys Leu Pro Lys Val Thr Arg
   1               5                   10
<210> 3
   <211> 12
   <212> PRT
   <213> Artificial Sequence
<220>
   <223> peptide identified by phage display panning on MDA-MB-231 cells
<400> 3
   Gly Lys Lys Gln Arg Phe Arg His Arg Asn Arg Lys
   1               5                   10
<210> 4
   <211> 12
   <212> PRT
   <213> Artificial Sequence
<220>
   <223> peptide identified by phage display panning on MDA-MB-231 cells
<400> 4
   Phe His Arg Arg Ile Lys Ala Gly Arg Gly Asp Ser
   1               5                   10
<210> 5
   <211> 12
   <212> PRT
   <213> Artificial Sequence
<220>
   <223> peptide identified by phage display panning on MDA-MB-231 cells
<400> 5
   Pro Gln Val Thr Arg Gly Asp Val Phe Thr Met Pro
   1               5                   10
<210> 6
   <211> 12
   <212> PRT
   <213> Artificial Sequence
<220>
   <223> peptide identified by phage display panning on MDA-MB-231 cells
<400> 6
   Leu Thr Gly Lys Asn Phe Pro Met Phe His Arg Asn
   1               5                   10
<210> 7
   <211> 12
   <212> PRT
   <213> Artificial Sequence
<220>
   <223> peptide identified by phage display panning on MDA-MB-231 cells
<400> 7
   Met His Arg Met Pro Ser Phe Leu Pro Thr Thr Leu
   1               5                   10
<210> 8
   <211> 11
   <212> PRT
   <213> Artificial Sequence
<220>
   <223> peptide identified by phage display panning on MDA-MB-231 cells
<400> 8
   Gly Trp Gln Pro Pro Ala Arg Ala Arg Ile Gly
   1               5                   10
<210> 9
   <211> 7
   <212> PRT
   <213> Artificial Sequence
<220>
   <223> peptide identified by phage display panning on MDA-MB-231 cells
<400> 9
   Thr Tyr Lys Tyr Tyr Pro Leu
   1               5
<210> 10
   <211> 7
   <212> PRT
   <213> Artificial Sequence
<220>
   <223> peptide identified by phage display panning on MDA-MB-231 cells
<400> 10
   His Ala Ile Tyr Pro Arg His
   1               5
<210> 11
   <211> 7
   <212> PRT
   <213> Artificial Sequence
<220>
   <223> peptide identified by phage display panning on MDA-MB-231 cells
<400> 11
   Gln Pro Pro Arg Ser Thr Ser
   1               5
<210> 12
   <211> 7
   <212> PRT
   <213> Artificial Sequence
<220>
   <223> peptide identified by phage display panning on MDA-MB-231 cells
<400> 12
   Ser Thr Ala Ser Tyr Thr Arg
   1 5
<210> 13
   <211> 7
   <212> PRT
   <213> Artificial Sequence
<220>
   <223> peptide identified by phage display panning on MDA-MB-231 cells
<400> 13
   Gly Lys Pro Met Pro Pro Met
   1 5
<210> 14
   <211> 7
   <212> PRT
   <213> Artificial Sequence
<220>
   <223> peptide identified by phage display panning on MDA-MB-231 cells
<400> 14
   Gln Pro Trp Pro Thr Ser Ile
   1 5
<210> 15
   <211> 7
   <212> PRT
   <213> Artificial Sequence
<220>
   <223> peptide identified by phage display panning on MDA-MB-231 cells
<400> 15
   His Thr Ile Gln Phe Thr Pro
   1 5
<210> 16
   <211> 7
   <212> PRT
   <213> Artificial Sequence
<220>
   <223> peptide identified by phage display panning on MDA-MB-231 cells
<400> 16
   Gln Pro Ser Met Leu Asn Pro
   1 5
<210> 17
   <211> 7
   <212> PRT
   <213> Artificial Sequence
<220>
   <223> peptide identified by phage display panning on MDA-MB-231 cells
<400> 17
   Gly Glu Thr Arg Ala Pro Leu
   1 5
<210> 18
   <211> 7
   <212> PRT
   <213> Artificial Sequence
<220>
   <223> peptide identified by phage display panning on MDA-MB-231 cells
<400> 18
   Ser Trp Gln Tyr Gly Lys Leu
   1 5
<210> 19
   <211> 7
   <212> PRT
   <213> Artificial Sequence
<220>
   <223> peptide identified by phage display panning on MDA-MB-231 cells
<400> 19
   Thr Tyr Arg Phe Gly Pro Leu
   1 5
<210> 20
   <211> 7
   <212> PRT
   <213> Artificial Sequence
<220>
   <223> peptide identified by phage display panning on MDA-MB-231 cells
<400> 20
   His Trp Lys Tyr Trp Pro Leu
   1 5
<210> 21
   <211> 7
   <212> PRT
   <213> Artificial Sequence
<220>
   <223> peptide identified by phage display panning on MDA-MB-231 cells
<400> 21
   Asp Leu Thr Val Thr Pro Trp
   1 5
<210> 22
   <211> 7
   <212> PRT
   <213> Artificial Sequence
<220>
   <223> peptide identified by phage display panning on MDA-MB-231 cells
<400> 22
   Leu Glu Val Phe Pro Tyr Tyr
   1 5
<210> 23
   <211> 7
   <212> PRT
   <213> Artificial Sequence
<220>
   <223> peptide identified by phage display panning on MDA-MB-231 cells
<400> 23
   Arg Ile Trp Asp Pro Pro Arg
   1 5
<210> 24
   <211> 5
   <212> PRT
   <213> Artificial Sequence
<220>
   <223> peptide identified by phage display panning on MDA-MB-231 cells
<400> 24
   Gly Arg Gly Asp Ser
   1 5
<210> 25
   <211> 5
   <212> PRT
   <213> Artificial Sequence
<220>
   <223> peptide identified by phage display panning on MDA-MB-231 cells
<400> 25
   Gly Gly Arg Asp Ser
   1 5
<210> 26
   <211> 7
   <212> PRT
   <213> Artificial Sequence
<220>
   <223> peptide identified by phage display panning on MDA-MB-231 cells
<400> 26
   Thr Ser Ser Glu Ser Glu Ser
   1 5
<210> 27
   <211> 7
   <212> PRT
   <213> Artificial Sequence
<220>
   <223> peptide identified by phage display panning on MDA-MB-231 cells
<400> 27
   Glu Arg Thr Val Leu His Thr
   1 5
<210> 28
   <211> 7
   <212> PRT
   <213> Artificial Sequence
<220>
   <223> peptide identified by phage display panning on MDA-MB-231 cells
<400> 28
   Arg Phe Thr Val Asp Trp Asp
   1 5
<210> 29
   <211> 7
   <212> PRT
   <213> Artificial Sequence
<220>
   <223> peptide identified by phage display panning on MDA-MB-231 cells
<400> 29
   Thr Leu Thr Val Gln Ala Trp
   1 5
<210> 30
   <211> 7
   <212> PRT
   <213> Artificial Sequence
<220>
   <223> peptide identified by phage display panning on MDA-MB-231 cells
<400> 30
   Leu Ala Gly Pro Leu Met Thr
   1 5
<210> 31
   <211> 7
   <212> PRT
   <213> Artificial Sequence
<220>
   <223> peptide identified by phage display panning on MDA-MB-231 cells
<400> 31
   Tyr Leu Thr Met Pro Thr Pro
   1 5
<210> 32
   <211> 7
   <212> PRT
   <213> Artificial Sequence
<220>
   <223> peptide identified by phage display panning on MDA-MB-231 cells
<400> 32
   Thr Pro Gln Ser Ser Pro Thr
   1 5
<210> 33
   <211> 7
   <212> PRT
   <213> Artificial Sequence
<220>
   <223> peptide identified by phage display panning on MDA-MB-231 cells
<400> 33
   Gly Val Lys Ala Leu Ser Thr
   1 5
<210> 34
   <211> 7
   <212> PRT
   <213> Artificial Sequence
<220>
   <223> peptide identified by phage display panning on MDA-MB-231 cells
<400> 34
   Thr Pro Phe Met Ala Tyr His
   1 5
<210> 35
   <211> 7
   <212> PRT
   <213> Artificial Sequence
<220>
   <223> peptide identified by phage display panning on MDA-MB-231 cells
<400> 35
   Ile Pro Ala Pro Leu Arg Ser
   1               5
<210> 36
   <211> 7
   <212> PRT
   <213> Artificial Sequence
<220>
   <223> peptide identified by phage display panning on MDA-MB-231 cells
<400> 36
   Glu Gln Gly Arg Pro Leu Pro
   1               5
<210> 37
   <211> 7
   <212> PRT
   <213> Artificial Sequence
<220>
   <223> peptide identified by phage display panning on MDA-MB-231 cells
<400> 37
   Met Ala Ala Asn Gly Ala Arg
   1               5
<210> 38
   <211> 7
   <212> PRT
   <213> Artificial Sequence
<220>
   <223> peptide identified by phage display panning on MDA-MB-231 cells
<400> 38
   Gln Val Leu Leu Thr Ala Ala
   1               5
<210> 39
   <211> 7
   <212> PRT
   <213> Artificial Sequence
<220>
   <223> peptide identified by phage display panning on MDA-MB-231 cells
<400> 39
   Ala Arg Ala Val Leu Gln Leu
   1 5
<210> 40
   <211> 7
   <212> PRT
   <213> Artificial Sequence
<220>
   <223> peptide identified by phage display panning on MDA-MB-231 cells
<400> 40
   Gln Asn Met Gln Gln Gln Ile
   1 5
<210> 41
   <211> 7
   <212> PRT
   <213> Artificial Sequence
<220>
   <223> peptide identified by phage display panning on MDA-MB-231 cells
<400> 41
   Ala Trp Ser Ala Val Met Arg
   1 5
<210> 42
   <211> 7
   <212> PRT
   <213> Artificial Sequence
<220>
   <223> peptide identified by phage display panning on MDA-MB-231 cells
<400> 42
   Asn Gln Leu Ala Gly Ser Gly
   1 5
<210> 43
   <211> 7
   <212> PRT
   <213> Artificial Sequence
<220>
   <223> peptide identified by phage display panning on MDA-MB-231 cells
<400> 43
   Thr Tyr Lys Phe Gly Thr Leu
   1 5
<210> 44
   <211> 7
   <212> PRT
   <213> Artificial Sequence
<220>
   <223> peptide identified by phage display panning on MDA-MB-231 cells
<400> 44
   His Trp His Phe Gly Pro Leu
   1 5
<210> 45
   <211> 7
   <212> PRT
   <213> Artificial Sequence
<220>
   <223> peptide identified by phage display panning on MDA-MB-231 cells
<400> 45
   Ser Trp Lys Phe Gly Pro Leu
   1 5
<210> 46
   <211> 7
   <212> PRT
   <213> Artificial Sequence
<220>
   <223> peptide identified by phage display panning on MDA-MB-231 cells
<400> 46
   Thr Trp Lys Phe Ser Pro Leu
   1 5
<210> 47
   <211> 7
   <212> PRT
   <213> Artificial Sequence
<220>
   <223> peptide identified by phage display panning on MDA-MB-231 cells
<400> 47
   Asn Val Ser Gly Ser His Ser
   1 5
<210> 48
   <211> 7
   <212> PRT
   <213> Artificial Sequence
<220>
   <223> peptide identified by phage display panning on MDA-MB-231 cells
<400> 48
   Ser Val Leu Leu Pro His Arg
   1 5
<210> 49
   <211> 7
   <212> PRT
   <213> Artificial Sequence
<220>
   <223> peptide identified by phage display panning on MDA-MB-231 cells
<400> 49
   Asp Ala Gly Gln Val Ser Gln
   1 5
<210> 50
   <211> 7
   <212> PRT
   <213> Artificial Sequence
<220>
   <223> peptide identified by phage display panning on MDA-MB-231 cells
<400> 50
   Arg Leu Pro Ser Trp His Glu
   1 5
<210> 51
   <211> 7
   <212> PRT
   <213> Artificial Sequence
<220>
   <223> peptide identified by phage display panning on MDA-MB-231 cells
<400> 51
   Ala Tyr Pro Glu Pro Tyr Val
   1 5
<210> 52
   <211> 7
   <212> PRT
   <213> Artificial Sequence
<220>
   <223> peptide identified by phage display panning on MDA-MB-231 cells
<400> 52
   Gln Pro Thr His Pro Thr Arg
   1 5
<210> 53
   <211> 7
   <212> PRT
   <213> Artificial Sequence
<220>
   <223> peptide identified by phage display panning on MDA-MB-231 cells
<400> 53
   Ala Pro Ile Trp Met His Val
   1 5
<210> 54
   <211> 7
   <212> PRT
   <213> Artificial Sequence
<220>
   <223> peptide identified by phage display panning on MDA-MB-231 cells
<400> 54
   Ala Thr Trp Gln Leu Gly Thr
   1 5
<210> 55
   <211> 7
   <212> PRT
   <213> Artificial Sequence
<220>
   <223> peptide identified by phage display panning on MDA-MB-231 cells
<400> 55
   Leu His Arg Gln Ser Ser Ala
   1 5
<210> 56
   <211> 7
   <212> PRT
   <213> Artificial Sequence
<220>
   <223> peptide identified by phage display panning on MDA-MB-231 cells
<400> 56
   Ala Ser Trp Ile Pro Leu Pro
   1 5
<210> 57
   <211> 7
   <212> PRT
   <213> Artificial Sequence
<220>
   <223> peptide identified by phage display panning on MDA-MB-231 cells
<400> 57
   Gln Gln Gln Tyr Met Ala His
   1 5
<210> 58
   <211> 7
   <212> PRT
   <213> Artificial Sequence
<220>
   <223> peptide identified by phage display panning on MDA-MB-231 cells
<400> 58
   Ser Thr Pro Ala Thr Leu Ile
   1 5
<210> 59
   <211> 7
   <212> PRT
   <213> Artificial Sequence
<220>
   <223> peptide identified by phage display panning on MDA-MB-231 cells
<400> 59
   Trp Ser Leu Ser Glu Leu His
   1 5
<210> 60
   <211> 7
   <212> PRT
   <213> Artificial Sequence
<220>
   <223> peptide identified by phage display panning on MDA-MB-231 cells
<400> 60
   Leu Pro Val Arg Leu Asp Trp
   1 5
<210> 61
   <211> 7
   <212> PRT
   <213> Artificial Sequence
<220>
   <223> peptide identified by phage display panning on MDA-MB-231 cells
<400> 61
   Gln Thr Trp Leu Glu Met Gly
   1 5
<210> 62
   <211> 7
   <212> PRT
   <213> Artificial Sequence
<220>
   <223> peptide identified by phage display panning on MDA-MB-231 cells
<400> 62
   Gly Pro His Asn Pro Thr Gln
   1 5
<210> 63
   <211> 7
   <212> PRT
   <213> Artificial Sequence
<220>
   <223> peptide identified by phage display panning on MDA-MB-231 cells
<400> 63
   Asn Asp Arg Pro His Met Pro
   1 5
<210> 64
   <211> 7
   <212> PRT
   <213> Artificial Sequence
<220>
   <223> peptide identified by phage display panning on MDA-MB-231 cells
<400> 64
   Val Pro Asn Ile Val Thr Gln
   1 5
<210> 65
   <211> 7
   <212> PRT
   <213> Artificial Sequence
<220>
   <223> peptide identified by phage display panning on MDA-MB-231 cells
<400> 65
   Ala Gly Ser Val Ile Asp Thr
   1 5
<210> 66
   <211> 7
   <212> PRT
   <213> Artificial Sequence
<220>
   <223> peptide identified by phage display panning on MDA-MB-231 cells
<400> 66
   Gln Ala Tyr His Val Ser Ala
   1 5
<210> 67
   <211> 7
   <212> PRT
   <213> Artificial Sequence
<220>
   <223> peptide identified by phage display panning on MDA-MB-231 cells
<400> 67
   Ser Asn Met Thr Arg Trp His
   1 5
<210> 68
   <211> 7
   <212> PRT
   <213> Artificial Sequence
<220>
   <223> peptide identified by phage display panning on MDA-MB-231 cells
<400> 68
   Gly Arg Leu Asp Thr Gly Ile
   1 5
<210> 69
   <211> 7
   <212> PRT
   <213> Artificial Sequence
<220>
   <223> peptide identified by phage display panning on MDA-MB-231 cells
<400> 69
   Ala Leu Gln Pro Gln Lys His
   1 5
<210> 70
   <211> 7
   <212> PRT
   <213> Artificial Sequence
<220>
   <223> peptide identified by phage display panning on MDA-MB-231 cells
<400> 70
   Ala Ser Tyr Ser Gly Thr Ala
   1 5
<210> 71
   <211> 7
   <212> PRT
   <213> Artificial Sequence
<220>
   <223> peptide identified by phage display panning on MDA-MB-231 cells
<400> 71
   Ala Thr Leu Thr His Pro Pro
   1 5
<210> 72
   <211> 7
   <212> PRT
   <213> Artificial Sequence
<220>
   <223> peptide identified by phage display panning on MDA-MB-231 cells
<400> 72
   Phe Pro Ser Thr Ile Thr Pro
   1 5
<210> 73
   <211> 7
   <212> PRT
   <213> Artificial Sequence
<220>
   <223> peptide identified by phage display panning on MDA-MB-231 cells
<400> 73
   His Pro Phe Glu His Phe Ser
   1 5
<210> 74
   <211> 7
   <212> PRT
   <213> Artificial Sequence
<220>
   <223> peptide identified by phage display panning on MDA-MB-231 cells
<400> 74
   Ile Pro Thr Leu Pro Ser Ser
   1 5
<210> 75
   <211> 7
   <212> PRT
   <213> Artificial Sequence
<220>
   <223> peptide identified by phage display panning on MDA-MB-231 cells
<400> 75
   Ser Ile Leu Pro Tyr Pro Tyr
   1 5
<210> 76
   <211> 7
   <212> PRT
   <213> Artificial Sequence
<220>
   <223> peptide identified by phage display panning on MDA-MB-231 cells
<400> 76
   Ser Thr Phe Thr Lys Ser Pro
   1 5
<210> 77
   <211> 7
   <212> PRT
   <213> Artificial Sequence
<220>
   <223> peptide identified by phage display panning on MDA-MB-231 cells
<400> 77
   Val Thr Ala His Gly Gly Arg
   1 5
<210> 78
   <211> 7
   <212> PRT
   <213> Artificial Sequence
<220>
   <223> peptide identified by phage display panning on MDA-MB-231 cells
<400> 78
   His Ala Leu Gly Pro Ser Ser
   1 5
<210> 79
   <211> 7
   <212> PRT
   <213> Artificial Sequence
<220>
   <223> peptide identified by phage display panning on MDA-MB-231 cells
<400> 79
   His His Ser Leu Thr Val Thr
   1 5
<210> 80
   <211> 7
   <212> PRT
   <213> Artificial Sequence
<220>
   <223> peptide identified by phage display panning on MDA-MB-231 cells
<400> 80
   Lys Ala Val His Pro Leu Arg
   1               5
<210> 81
   <211> 7
   <212> PRT
   <213> Artificial Sequence
<220>
   <223> peptide identified by phage display panning on MDA-MB-231 cells
<400> 81
   Ser Phe Val Leu Pro Tyr Tyr
   1               5
<210> 82
   <211> 7
   <212> PRT
   <213> Artificial Sequence
<220>
   <223> peptide identified by phage display panning on MDA-MB-231 cells
<400> 82
   Ser His Thr Ala Pro Leu Arg
   1               5
<210> 83
   <211> 7
   <212> PRT
   <213> Artificial Sequence
<220>
   <223> peptide identified by phage display panning on MDA-MB-231 cells
<400> 83
   Ser Pro Thr Gln Pro Lys Ser
   1               5
<210> 84
   <211> 7
   <212> PRT
   <213> Artificial Sequence
<220>
   <223> peptide identified by phage display panning on MDA-MB-231 cells
<400> 84
   Ser Ser Leu Val Arg Thr Ala
   1 5
<210> 85
   <211> 7
   <212> PRT
   <213> Artificial Sequence
<220>
   <223> peptide identified by phage display panning on MDA-MB-231 cells
<400> 85
   Thr Ala Arg Tyr Pro Ser Trp
   1 5
<210> 86
   <211> 7
   <212> PRT
   <213> Artificial Sequence
<220>
   <223> peptide identified by phage display panning on MDA-MB-231 cells
<400> 86
   Thr Phe Ala Lys Ser Ala Tyr
   1 5
<210> 87
   <211> 7
   <212> PRT
   <213> Artificial Sequence
<220>
   <223> peptide identified by phage display panning on MDA-MB-231 cells
<400> 87
   Thr Pro Pro Thr Met Asp His
   1 5
<210> 88
   <211> 7
   <212> PRT
   <213> Artificial Sequence
<220>
   <223> peptide identified by phage display panning on MDA-MB-231 cells
<400> 88
   Val Ile Pro His Val Leu Ser
   1 5
<210> 89
   <211> 7
   <212> PRT
   <213> Artificial Sequence
<220>
   <223> peptide identified by phage display panning on MDA-MB-231 cells
<400> 89
   Trp Thr Ile Thr Lys His Pro
   1 5
<210> 90
   <211> 7
   <212> PRT
   <213> Artificial Sequence
<220>
   <223> peptide identified by phage display panning on MDA-MB-231 cells
<400> 90
   Tyr Ala Gly Pro Tyr Gln His
   1 5
<210> 91
   <211> 7
   <212> PRT
   <213> Artificial Sequence
<220>
   <223> peptide identified by phage display panning on MDA-MB-231 cells
<400> 91
   Tyr Lys His Pro Pro Gln His
   1 5
<210> 92
   <211> 7
   <212> PRT
   <213> Artificial Sequence
<220>
   <223> peptide identified by phage display panning on MDA-MB-231 cells
<400> 92
   Ala Met Ser Ser Arg Ser Leu
   1 5
<210> 93
   <211> 7
   <212> PRT
   <213> Artificial Sequence
<220>
   <223> peptide identified by phage display panning on MDA-MB-231 cells
<400> 93
   Met Asp Ala His His Ala Leu
   1 5
<210> 94
   <211> 7
   <212> PRT
   <213> Artificial Sequence
<220>
   <223> peptide identified by phage display panning on MDA-MB-231 cells
<400> 94
   Asn His Trp Ala Ser Pro Arg
   1 5
<210> 95
   <211> 7
   <212> PRT
   <213> Artificial Sequence
<220>
   <223> peptide identified by phage display panning on MDA-MB-231 cells
<400> 95
   Ser Thr Ile His Gly Ser Thr
   1 5
<210> 96
   <211> 7
   <212> PRT
   <213> Artificial Sequence
<220>
   <223> peptide identified by phage display panning on MDA-MB-231 cells
<400> 96
   Asp Ser His Thr Pro Gln Arg
   1 5
<210> 97
   <211> 7
   <212> PRT
   <213> Artificial Sequence
<220>
   <223> peptide identified by phage display panning on MDA-MB-231 cells
<400> 97
   Asn Pro Pro Ser Arg His Pro
   1 5
<210> 98
   <211> 7
   <212> PRT
   <213> Artificial Sequence
<220>
   <223> peptide identified by phage display panning on MDA-MB-231 cells
<400> 98
   Asn Gln Leu Pro Leu His Ala
   1 5
<210> 99
   <211> 7
   <212> PRT
   <213> Artificial Sequence
<220>
   <223> peptide identified by phage display panning on MDA-MB-231 cells
<400> 99
   Ser His Ala Leu Arg Thr Val
   1 5
<210> 100
   <211> 7
   <212> PRT
   <213> Artificial Sequence
<220>
   <223> peptide identified by phage display panning on MDA-MB-231 cells
<400> 100
   Ser Leu Pro Phe Gln Leu Thr
   1 5
<210> 101
   <211> 7
   <212> PRT
   <213> Artificial Sequence
<220>
   <223> peptide identified by phage display panning on MDA-MB-231 cells
<400> 101
   Ser Pro Gln Met Thr Leu Ser
   1 5